# EUROPÄISCHE PATENTSCHRIFT

(11) **EP 3 326 586 B1**
(45) Veröffentlichungstag und Bekanntmachung des Hinweises auf die Patenterteilung: **27.05.2020**
(21) Anmeldenummer: 17204574.2
(22) Anmeldetag: 18.11.2014
(51) Int. Cl.: A61F 2/36, A61F 2/40, A61F 2/38, A61F 2/42, A61F 2/34, A61F 2/30

(54) **BESCHICHTETES HEMIPROTHESEN-IMPLANTAT**
COATED HEMIPROSTHESIS IMPLANT
IMPLANT À HÉMIPROTHÈSE REVÊTUE

(30) Priorität: 23.12.2013 DE 102013227136
(43) Veröffentlichungstag der Anmeldung: 30.05.2018
(62) Teilanmeldung aus: 14808527.7
(73) Patentinhaber: Mathys AG Bettlach, 2544 Bettlach (CH)
(72) Erfinder: Lerf, Reto, 4513 Langendorf (CH); Scheurer, Stefan, 4571 Lüterkofen (CH)
(74) Vertreter: Körfer, Thomas

(56) Entgegenhaltungen:
- EP-A1- 0 608 997
- DE-U1-202008 015 356
- US-A1- 2007 299 520
- US-B1- 6 709 463

## Beschreibung

Die vorliegende Erfindung betrifft ein Hemiprothesen-Implantat, einen Implantatsatz umfassend ein Hemiprothesen-Implantat sowie ein Verfahren zur Herstellung eines Hemiprothesen-Implantats.

Implantate sind künstliche, d.h. nicht-natürliche Strukturen, die in den menschlichen oder tierischen Körper implantiert werden, etwa um dort in Form einer Endoprothese ein Gelenk ganz oder teilweise zu ersetzen. Bei den betroffenen Gelenken kann es sich bspw. um Schulter- oder Hüftgelenk, Knie, oder Sprunggelenk handeln.

Ein Gelenk besteht im Grundsatz aus zwei Knochenenden, d.h. Gelenkhälften, die einander mit den jeweiligen Gelenk- oder Gleitflächen beweglich gegenüber stehen. Bei Kugelgelenken wie etwa Schulter- oder Hüftgelenk hat ein Gelenkteil eine konkave Gleitfläche, etwa das Glenoid bzw. die Gelenkpfanne beim Schultergelenk oder das Acetabulum bzw. die Gelenkpfanne beim Hüftgelenk, während die andere Gleitfläche der Gleitpaarung konvex ausgebildet ist, etwa der Humeruskopf bzw. Oberarmkopf beim Schultergelenk oder der Hüft- bzw. Oberschenkelkopf beim Hüftgelenk.

Beim Gelenkersatz ist zwischen Total- oder Hemiprothese zu unterscheiden. Eine Totalprothese ersetzt das gesamte Gelenk, d.h. jede der beiden Gelenkhälften wird durch ein Implantat ersetzt, wobei das Implantat im verbleibenden Knochen verankert wird. Die Gleitpaarung zwischen dem konvexen und dem konkaven Gelenkersatzteil wird ausschließlich durch künstliche Materialien vermittelt.

Eine Hemiprothese ersetzt nur eine Gelenkhälfte, meist den konvexen Gleitpartner. Verglichen mit einer Totalprothese ist der Eingriff weniger schwerwiegend und die Belastung für den Patienten geringer. Eine Hemiprothese kann bspw. dann gewählt werden, wenn der Knorpel der natürlichen konkaven Gleitfläche von Glenoid, Acetabulum, oder auch Tibiaplateau beim Knie, etc. gesund und intakt ist. Beim Hüftgelenk werden z.B. bei beschädigtem Hüftkopf oder Fraktur des Schenkelhalses diese beiden Teile des Femurknochens ersetzt, aber beckenseitig das Acetabulum belassen.

Gelenkersatzteile müssen ausreichend standfest sein, um dauerhaft die an Gelenken wirkenden Kräfte übertragen zu können. Deshalb werden Implantate in diesem Bereich häufig aus Metall oder Keramik gefertigt. Bei Hemiprothesen ergibt sich jedoch das Problem, dass standfeste Prothesenmaterialien keine guten Reibpartner für den natürlichen Knorpel der anderen Gelenkhälfte sind. So ist es bekannt, dass etwa Stahl oder Kobaltlegierungen zu Schäden führen, die bis zur Zerstörung des Knorpels reichen können.

Es werden verschiedene Materialien vorgeschlagen, die anstelle von Metall oder Keramik als Gleitpartner für Knorpel verwendet werden sollen. Es liegen bisher nur von einigen dieser Materialien Ergebnisse bzgl. ihrer tatsächlichen Eignung vor. Jedoch kann man die Materialien im Vergleich mit Metall oder Keramik grob in steife bzw. harte und weniger steife bzw. weniger harte Materialien einteilen. Beispiele für harte Materialien sind etwa oxidkeramisch beschichtete Metalle wie etwa oxidiertes Zirkon, Oxidkeramiken, pyrolitischer Kohlenstoff (Pyrocarbon).

Beispiele für tendenziell weniger steife Materialien umfassen verschiedene Kunststoffe. Zumindest einige dieser Materialien sind nicht standfest genug, um sie als Grundwerkstoff zur Herstellung von Implantaten wie Gelenkersatzteilen einzusetzen. Ein entsprechendes Implantat muss daher zweiteilig aufgebaut sein, d.h. eine standfestes Material mit einer Beschichtung aus einem knorpelschonenden Material umfassen. Problematisch bleibt jedoch, wie ein steifes Grundmaterial mit einem weniger steifen Beschichtungsmaterial im Hinblick auf die in einem Gelenk wirkenden Kräfte dauerhaft und untrennbar gefügt werden kann.

Aus der EP 2 086 471 B1 ist es bekannt, eine Pyrocarbon-Schale auf einem metallischen Träger zu fixieren. Hierbei ist eine Polyethylen-Zwischenschicht vorgesehen. Die Pyrocarbon-Schale ist mit einer einwärts gerichteten Basiskante ausgebildet, die in eine entsprechende, umlaufende Aussparung der Polyethylen-Zwischenschicht eingreift, so dass sich ein Preßsitz ergibt. Die Polyethylen-Auskleidung und der metallische Träger verfügen jeweils über eine korrespondierende umlaufende Nut, in die ein ringförmiger Halterungsring aufgenommen ist. Die Anordnung kann sich zur Befestigung von steifen Materialien wie Pyrocarbon eignen, ist aber nicht auf andere Materialien geringerer Steifigkeit übertragbar.

Aus der US 6,709,463 B1 ist ein Implantat nach dem Oberbegriff des Anspruchs 1 bekannt.

Eine Aufgabe der vorliegenden Erfindung besteht darin, eine Hemiprothese zu schaffen, bei der ein Implantat aus einem steifen Grundmaterial aufgebaut ist, eine Artikulationsfläche jedoch von einem weniger steifen Material gebildet wird, und bei der eine dauerhafte und sichere Fügung des weniger steifen Materials auf das Grundmaterial u.a. auch im Hinblick auf Kostengesichtspunkte optimiert ist.

Diese Aufgabe wird durch ein Hemiprothesen-Implantat mit den Merkmalen des Anspruchs 1, einen Implantatsatz mit den Merkmalen des Anspruchs 12, sowie ein Verfahren zur Herstellung eines Hemiprothesen-Implantats mit den Merkmalen des Anspruchs 13 gelöst. Die davon abhängigen Ansprüche enthalten vorteilhafte Weiterbildungen.

Erfindungsgemäß wird ein Hemiprothesen-Implantat vorgeschlagen, welches ein Gelenkersatzteil mit einer Artikulationsoberfläche zur Gleitpaarung mit einer Gelenkfläche eines natürlichen Gelenkgegenparts umfasst. Die Artikulationsoberfläche umfasst eine auf einem Untergrund aufgebrachte Beschichtung. Der Untergrund weist ein Relief zur Anhaftung der Beschichtung auf.

Bei einigen bevorzugten Ausführungsformen umfasst das Implantat als einzige Komponente das Gelenkersatzteil. Es kann sich dabei etwa um einen einstückig, z.B. als Monoblock, ausgeführten Prothesenschaft mit einem Kopfersatz handeln. Bei anderen Ausführungsformen umfasst das Implantat neben dem Gelenkersatzteil eine oder mehrere weitere Komponenten. Beispielsweise kann ein Implantat als Gelenkersatzteil einen Kopfersatz und als weitere separate Komponente einen Prothesenschaft umfassen.

Die Artikulationsfläche des Gelenkersatzteils kann zumindest bereichsweise konvex ausgebildet sein, um mit einer konkaven Gelenkfläche eines natürlichen Gelenkteils bzw. Gelenk-Gegenübers zu artikulieren.

Das Implantat, d.h. das Gelenkersatzteil und/oder weitere Komponenten können etwa einen Humerushemiprothesenkopf, einen Hüfthemiprothesenkopf, eine Femurhemiprothesenkomponente, bspw. einen Condylus-Gelenkersatz und/oder Meniskusersatz, oder eine Sprungbeinhemiprothesenrolle umfassen.

Der Untergrund für die Beschichtung kann durch ein Material eines Grundkörpers des Gelenkersatzteils gebildet sein. Besteht das Gelenkersatzteil z.B. im Hinblick auf eine Lastaufnahme und/oder Kraftübertragung im Wesentlichen aus einem Material wie einem Metall oder einer Keramik, kann dieses Material den Untergrund ausbilden. Bei einigen Ausführungsformen wird mindestens einer der folgenden Werkstoffe zur Herstellung des Grundkörpers verwendet: ein Metall wie etwa Titan, eine oder mehrere Titanlegierungen, Stahl, insb. rostfreier Stahl, eine oder mehrere Kobaltlegierungen, Zirkonium, eine oder mehrere Zirkoniumlegierungen, Tantal, eine oder mehrere Tantallegierungen, eine oder mehrere Molybdänlegierungen, eine Keramik wie etwa eine Aluminiumoxidkeramik, eine Zirkonoxidkeramik, eine oder mehrere Mischkeramiken wie etwa Aluminiumoxid-Zirkonoxid, ein Kunststoff, bspw. ein verstärkter Kunststoff wie etwa ein faserverstärkter Kunststoff.

Der Untergrund kann auch ganz oder teilweise durch ein anderes Material als das des Grundkörpers gebildet sein, bspw. kann das Material einer auf einem Grundkörper gebildeten Zwischenschicht den Untergrund bilden. Bei dem Material der Zwischenschicht kann es sich bspw. ebenfalls um ein steifes Material handeln, etwa um eines der oben genannten Werkstoffmaterialien und/oder ein anderes Material wie etwa Pyrocarbon. Zusätzlich oder alternativ kann das Material der Zwischenschicht auch einen anderen Kunststoff wie etwa ein Polyethylen umfassen, etwa wenn eine Zwischenschicht mehrere Unterschichten umfasst.

Im Hinblick auf seine Eigenschaft als Grundlage für eine Beschichtung kann auf den Untergrund auch mit den folgenden Begriffen Bezug genommen werden: Substrat, Fundament, Unterlage, Träger, Basis.

Die Beschichtung kann im Vergleich zu anderen Abmessungen des Gelenkersatzteils eine dünne Schicht auf dem Untergrund bilden. Die Dicke der Beschichtung kann bspw. weniger als 20%, oder weniger als 10%, oder weniger als 5%, eines Radius eines konvexen Gelenkersatzteils betragen. Die Beschichtung kann daher u.U. auch als Kappe, Mantel, Überzug auf dem Untergrund, Umhüllung, Ummantelung, Verkleidung, und/oder Verschalung angesehen werden.

Die Beschichtung kann ein Material oder mehrere Materialien aus der folgenden Liste umfassen: ein thermoplastisches Elastomer (TPE), ein Polycarbonaturethan (PCU), ein Polyolefin-Polymer, ein UHMWPE, ein XUHMWPE, ein UHMWPE oder XUHMWPE mit einem oder mehreren Additiven wie etwa Vitamin E, ein Polyarylketon bzw. Polyarylehterkethon, ein Polyetherketonketon (PEKK), ein Hydrogel, ein Hydrogel basierend auf PVA und/oder PVD.

Der Untergrund unter der Beschichtung kann strukturiert bzw. konturiert sein, so dass der Untergrund das Relief zur Anhaftung der Beschichtung aufweist. Statt vom Relief könnte auch von einem Terrain oder einer Topographie des Untergrundes die Rede sein. Das Relief bietet eine Struktur zum Angriff bzw. zur Anhaftung oder zum Halt für die Beschichtung. Unter einem Relief kann vorliegend jegliche Oberflächenvergrößerung des Untergrundes verstanden werden, um eine stabile Verankerung einer, bspw. gespritzten Beschichtung bzw. Kappe zu erreichen.

Das Relief kann eine Struktur aufweisen, die gegenüber einer Grundfläche abgesenkt oder versenkt ist. Bspw. kann das Relief Aussparungen oder Vertiefungen umfassen. Eine Tiefe derartiger Aussparungen kann etwa vergleichbar zu oder kleiner als eine Dicke der Beschichtung sein. Bspw. kann eine maximale Tiefe der Aussparungen eines Reliefs 100% oder weniger einer Beschichtungsdicke betragen, oder 50% oder weniger, oder 30% oder weniger, oder 10% oder weniger.

Eine Aussparung oder Vertiefung im Relief kann lokal oder punktuell ausgebildet sein, bspw. in Form einer Grube oder Mulde, Hinterschneidung, etc. Erfindungsgemäß sind punktuelle Vertiefungen gemäß globalen oder großräumigen Mustern auf der Artikulationsfläche angeordnet. Bspw. können punktuelle Vertiefungen linear, d.h. entlang von Linien, und/oder flächig zur Ausbildung von Bereichen mit einer Vielzahl von Vertiefungen angeordnet werden.

Zusätzlich oder alternativ können lineare Aussparungen ausgebildet sein, etwa in Form einer Mehrzahl von Nuten, Riefen, Rinnen, Gräben oder Rillen, ggf. mit Hinterschneidungen. Die Aussparungen können lokal geradeaus verlaufen, wobei sich bei konvexen Artikulationsflächen bspw. Großkreisen um einen Kopfbereich des Gelenkersatzteiles herum ergeben können. Andere Aussparungen können eine lokale Krümmung aufweisen und können etwa Teil- oder Vollkreise beschreiben, Schlangenlinien, etc.

Zusätzlich oder alternativ können flächige Aussparungen ausgebildet sein. So können etwa einer Mehrzahl abgesenkter Bereiche vorliegen. Bspw. können rechteckförmig, quadratisch, kreisförmig, und/oder halbmondförmig abgesenkte Bereiche vorliegen.

Bei manchen Ausführungsformen kann das Relief mindestens zwei sich kreuzende Nute aufweisen. Kreuzungswinkel zwischen sich kreuzenden Nuten können spitz sein, können also etwa 45° oder weniger betragen, und/oder können stumpf sein, also etwa mehr als 45° betragen. Bei verschiedenen Ausführungsformen umfasst das Relief eine Mehrzahl von Längs- und Quernuten, wobei sich jeweils eine Längs- und eine Quernut unter einem Winkel von 80° oder mehr schneiden.

Durch sich regelmäßig kreuzende Nute, bspw. gitterartig angeordnete Nute und/oder andere Arten linearer Vertiefungen können eine Vielzahl erhabener Bereiche definiert werden, die bspw. angenähert quadratisch, rechteckförmig, rautenförmig, oder in anderer Weise segmentförmig sein können. Auf diese Weise kann etwa eine Riffelung des Untergrundes ausgebildet werden.

Bei bestimmten Ausführungsformen umfasst das Relief einen aufgerauten Untergrundbereich oder mehrere derartiger Bereiche. Eine Aufrauhung kann ein unregelmäßiges Muster in den Untergrund einbringen. Eine bereichsweise Aufrauhung kann mehrfach vorliegen, bspw. können eine Vielzahl gleichartig aufgerauter Bereiche in einem schachbrettartigen Muster vorliegen, oder entlang eines Großkreises angeordnet sein, etc. Die Rauigkeit kann entsprechend ausgeprägt sein wie dies oben für die Tiefe von Aussparungen skizziert wurde.

Zusätzlich oder alternativ zu Vertiefungen oder Aufrauhungen kann das Relief eine Struktur aufweisen, die gegenüber einer Grundfläche erhöht oder erhaben ist. Es wird angemerkt, dass etwa bei vielfältig strukturierten Reliefs die Definition einer Grundfläche und damit die Definition einer Struktur als Vertiefung oder Erhebung willkürlich sein können.

Erhabene Bereiche oder Erhebungen im Relief können lokal oder punktuell ausgebildet sein. Bspw. kann das Relief eine Vielzahl an Spitzen, Nocken, Zähnen, Zacken, Zinken, Zinnen, Buckeln, etc. aufweisen. Bei einigen Ausführungsformen können punktuelle Erhebungen gemäß globalen oder großräumigen Mustern angeordnet sein, bspw. linear oder flächig zur Ausbildung entsprechender Bereiche mit einer Vielzahl von Erhebungen.

Zusätzlich oder alternativ können Erhebungen linear ausgebildet sein, etwa in Form von lokal gerade oder gebogen verlaufenden Vorsprüngen, Auskragungen, Kanten, etc. Zusätzlich oder alternativ können Erhebungen flächig ausgebildet sein. Bspw. können rechteckförmig, quadratisch, kreisförmig, und/oder halbmondförmig erhabene Bereiche vorliegen.

Einige Ausführungsformen des Reliefs umfassen eine Mehrzahl linear angeordneter Erhebungen derart, dass auf der Substratoberfläche etwa eine Mehrzahl an Zahnreihen ausgebildet ist. Eine Zahnreihe kann bspw. durch Zähne, Zinken oder Zacken gebildet werden, die sich aus einer Grundfläche, oder einer erhabenen Kante oder sonstigen linearen Erhöhung heraus erheben und so eine Zahnung, Rändelung oder Ritzelung bilden.

Bei einigen dieser Ausführungsformen weist das Relief eine Mehrzahl von längs verlaufenden Zahnreihen auf; bspw. können derartige Zahnreihen bei einer kugelsegmentförmig oder anders abgerundeten konvexen Artikulationsfläche in Richtung von einer Spitze zu einer der Spitze gegenüberliegenden Basis eines Kopfersatzes oder sonstigen Begrenzung der Artikulationsfläche verlaufen. Zusätzlich oder alternativ können quer verlaufende Zahnreihen vorgesehen sein, Zahnreihen die in Zickzackform verlaufen, einander kreuzen, eine X-Form bilden, etc.

Bei einigen Ausführungsformen kann mindestens eine in den Untergrund hineinführende Entlüftungsbohrung vorliegen. Für die Entlüftungsbohrungen genügt es, dass sie einen Durchtritt bzw. ein Verdrängen bzw. Entweichen von Luft oder einer sonstigen bspw. während des Aufbringens der Beschichtung vorhandenen Atmosphäre ermöglichen. Dies kann generell gelten oder in Bereichen in denen ein seitliches Entweichen in bestimmte Richtungen behindert ist, also etwa in Vertiefungen wie bspw. in einer Nut. Die Entlüftungsbohrungen sollten nicht so groß sein, dass sie eine Anhaftfläche des Untergrundes merklich verringern und/oder einen Durchtritt einer aufgebrachten Beschichtung, bspw. eines Spritzgussmaterials, in einen Innenbereich des Grundkörpers zulassen.

Bohrungen können punktuell eingebracht werden, also bspw. als zylinderförmigen Bohrungen, oder können mit einer bevorzugten Längserstreckung eingebracht werden, also bspw. als Spalte oder Rinne. Bspw. kann entlang einer Nut bzw. am Boden einer Nut eine längliche Entlüftungsspalte verlaufen. Eine solche Spalte könnte im wesentlichen die Länge der Nut haben, oder es kann eine Mehrzahl von Entlüftungsspalten kürzerer Länge entlang der Nut eingebracht sein; zusätzlich oder alternativ kann eine Entlüftungsspalte durch Brücken oder Rippen unterteilt sein. Entlang des Bodens einer Nut können auch eine Mehrzahl kreisförmiger Bohrungen eingebracht sein, so dass der Boden der Nut in regelmäßigen Abständen punktiert ist.

Bohrungen können sich in den Grundkörper hinein erstrecken. Die Entlüftungsbohrungen können entweder blind enden oder können offen sein, d.h. den Grundkörper vollständig durchdringen, bspw. indem sie bis in eine zentrale oder innere Ausnehmung des Grundkörpers hineinreichen. Blind endende Entlüftungsbohrungen können ausreichen, sofern die gewünschte Entlüftungswirkung erzielt werden kann. Mischformen sind denkbar, so kann etwa eine Entlüftungsrinne oder -nut mit punktuellen offenen Bohrungen versehen sein, die in einem geschlossenen oder offenen Hohlraum des Grundkörpers münden.

Zusätzlich oder alternativ zu Entlüftungsbohrungen können Entlüftungsnute vorgesehen sein, die entlang des Untergrundes verlaufen. Wie für die Entlüftungsbohrungen gilt auch für die Entlüftungsnute, dass diese in Bezug auf ihre Tiefe, Breite, und/oder Länge klein sein sollten, und zwar im Vergleich zu den eigentlichen Anhaftungsstrukturen wie etwa den Verankerungsnuten, um eine Verankerungswirkung, d.h. eine Anhaftung der Beschichtung nicht unerwünscht zu beeinflussen.

Entlüftungsbohrungen und/oder -nute können unabhängig von Reliefstrukturen vorgesehen sein, d.h. es können Entlüftungsbohrungen oder -nute in einem Untergrundbereich des Prothesengrundkörpers vorgesehen sein, in dem kein Relief vorgesehen ist. Zusätzlich oder alternativ können Entlüftungsbohrungen oder -nute sich an Reliefstrukturen wie bspw. Vertiefungen, oder erhabenen Strukturen, orientieren. So können bspw. Entlüftungsnute entlang einer Vertiefung vorgesehen sein, bspw. können Entlüftungsnute in derartige Vertiefungen hineinführen bzw. von dort wegführen. Entlüftungsbohrungen oder -nute können aber auch benachbart zu Vorsprüngen wie bspw. Zähnen angelegt sein.

Es kann bspw. ein Netz oder ein Muster von einzelnen und/oder kreuzenden Entlüftungsnuten vorgesehen sein. Zum Beispiel kann in Zusammenhang mit einem aufgerauten Untergrundbereich ein Muster von Entlüftungsbohrungen und/oder -nuten vorgesehen sein. Alternativ oder zusätzlich kann in Zusammenhang mit einem planen Untergrundbereich ein Muster von Entlüftungsbohrungen und/oder -nuten vorgesehen sein. Es sind auch Ausführungsbeispiele von Hemiprothesenköpfen denkbar, die keinerlei Relief aufweisen, wohl aber Entlüftungsbohrungen und/oder -nute. Bspw. kann sich ein Netz oder Muster von Entlüftungsbohrungen und/oder -nuten über einen Teil des Untergrundes oder den gesamten Untergrund erstrecken, der von einer Beschichtung überdeckt wird, d.h. das Muster kann sich unterhalb und/oder außerhalb der Artikulationsfläche erstrecken.

Ob Entlüftungsbohrungen und/oder -nute vorgesehen werden und wie diese ausgelegt werden, kann im Einzelfall vom verwendeten Beschichtungsmaterial abhängen, von den Strukturen des Untergrundes wie etwa der Anzahl und Anordnung von Nuten, Zähnen, etc. Auch der Einsatzzweck kann zu berücksichtigen sein, also etwa ob es sich um großen oder kleinen Gelenkersatz handelt. Die erforderliche Verankerungswirkung kann auch durch eine angestrebte Lastaufnahme vorgegeben sein. Ggf. können Versuche durchgeführt werden, welche Entlüftungsstrukturen zu einer optimalen Anhaftung der Beschichtung auf dem Untergrund bzw. der Kappe auf dem Grundkörper führen.

Ein Grundkörper, der den Untergrund bildet, kann einen nach außen freiliegenden Sockel aufweisen. Bei einigen Ausführungsformen kann unmittelbar am Sockel eine mindestens teilweise umlaufende Verankerungsnut ausgeführt sein, die z.B. der Verankerung des Mantels bzw. der Kappe auf dem Grundkörper dient. Bei diesen oder anderen Ausführungsformen kann ein freiliegender Sockelbereich des den Untergrund bildenden Grundkörpers für den Übergang zur Artikulationsoberfläche scharfkantig ausgeführt sein, etwa um einen Fügespalt an der äußeren bzw. freiliegenden Grenze zwischen Kappe und Grundkörper zu minimieren.

Der Untergrund, auf dem ein Relief, eine Entlüftungsbohrung, etc. ausgebildet sein kann, umfasst den gesamten Untergrund auf dem eine Beschichtung, Kappe, etc. ausgebildet wird oder ist. Der Untergrund kann also eine Oberfläche des Prothesengrundkörpers umfassen, der sich unter der Artikulationsoberfläche befindet, kann aber auch Oberflächenbereiche des Grundkörpers umfassen, die nicht unterhalb der Artikulationsoberfläche liegen, sondern daneben bzw. außerhalb. Unter der verwendeten Formulierung, ein Relief befände sich "außerhalb" der Artikulationsoberfläche, ist zu verstehen, dass die über einem Relief liegende Oberfläche der Beschichtung bzw. Kappe nicht, oder zumindest nicht bei normalen Gelenkbewegungen, in Kontakt mit einem Gelenkgegenpart (bspw. Knorpel) steht, sondern bspw. mit dem einen Prothesenschaft umgebenden Knochen oder umliegendem Gewebe in Kontakt steht. Im allgemeinen hat die Beschichtung bzw. Kappe eine Oberfläche, die größer ist bzw. mehr umfasst als die Artikulationsoberfläche; dies bedeutet, dass im allgemeinen auch der Untergrund größer ist als die (normale) Artikulationsoberfläche.

Bei einigen Ausführungsformen ist ein Relief nur auf dem Untergrund oder auf Untergrundbereichen unterhalb der Artikulationsoberfläche ausgebildet. Bei anderen Ausführungsformen erstreckt sich das Relief auf einen Untergrundbereich, der nicht unter der Artikulationsoberfläche liegt. Bspw. können Untergrundbereiche unter und neben der Artikulationsoberfläche ein Relief oder mehrere Reliefs aufweisen. Bei manchen dieser Ausführungsformen kann sich das Relief zu einem wesentlichen Teil neben der Artikulationsoberfläche befinden.

Beispielsweise kann das Relief bei einem im wesentlichen sphärisch ausgebildeten Grundkörper einen Untergrundbereich an der Spitze freilassen, d.h. unter einem zentralen Bereich der Artikulationsoberfläche befindet sich kein Relief. Bei einem anderen Beispiel kann ein Bereich oberhalb eines Äquators freigelassen sein, d.h. in einen Bereich der bei normalen Bewegungsvorgängen nicht zur Artikulationszone gehört, befindet sich kein Relief.

Bei einigen Ausführungsformen ist unterhalb der Artikulationsoberfläche kein Relief vorhanden, d.h. dort ist der vom Prothesenkörper bereitgestellte Untergrund plan und ein Relief befindet sich ausschließlich außerhalb der (normalen) Artikulationszone, d.h. außerhalb der Zone, die im normalen Bewegungsumfang des Gelenks direkt gegen den (natürlichen) Gelenkgegenpart wie bspw. Knorpel artikuliert. Bei einer Hüftkugel wäre das bspw. die Zone unterhalb des Äquators, d.h. in Richtung auf den Prothesenschaft; bei einem Schulterkopf wäre das etwa die gegen den resektierten Humerus zeigende, ebene Fläche.

Wie großflächig und/oder in welchem Bereich ein Relief aufgebracht wird, kann etwa von der zu erzielenden Haftwirkung abhängen. Unter der Artikulationsoberfläche kann z.B. dann ganz oder teilweise auf ein Relief verzichtet werden, wenn die mit dem verbleibenden Relief erzielbare Verankerung der Kappe ausreichend ist. Bei Ausführungsformen, bei denen ganz oder teilweise auf ein Relief unterhalb der Artikulationsoberfläche verzichtet wird, kann ein Relief nur einen Sockelbereich des Grundkörpers umfassen, einen Bereich wie eine Kante oder Nut, in den die Kappe einrasten soll, etc.

Bei einem Ansatz kann durch eine Entlüftung mittels entsprechender Entlüftungsbohrungen und/oder -nuten eine ausreichende Haftwirkung erzeugt werden, so dass ein erfindungsgemäßes Relief sich nicht auf dem gesamten Untergrund unter einer Artikulationszone und/oder außerhalb davon erstreckt. Hierbei können Entlüftungsbohrungen und/oder - nute entweder im Bereich der Artikulationszone vorgesehen werden, auch wenn dort kein Relief vorliegt, und/oder es können Entlüftungsbohrungen und/oder -nute in Bereichen außerhalb der Artikulationszone vorgesehen werden, etwa in einem Bereich wo das Relief vorliegt, also zusammen mit dem Relief, und/oder in einem Bereich wo kein Relief vorliegt.

Einige der erwähnten Ausführungsformen bieten Optionen, um eine Abweichung des Untergrundes von einer Sollgeometrie im Bereich der Artikulationsoberfläche zu minimieren. Unter der Artikulationsfläche ganz oder teilweise auf ein Relief zu verzichten, kann bspw. abhängig von einem verwendeten Material für die Beschichtung vorteilhaft sein, wenn auf diese Weise Spritzfehler vermieden werden können wie bspw. Einfallstellen, deren Auftreten zu Abweichungen von der Sollgeometrie der Artikulationsoberfläche führen könnte.

Eine Abweichung von einer Sollgeometrie kann etwa auch aus Sicherheitsgründen gefordert sein, etwa aufgrund von gesetzlichen Bestimmungen oder auf Veranlassung einer medizinischen Aufsichtsbehörde, bspw. für jeglichen Gelenkersatz oder für Gelenkersatz mit besonders hoher Lastaufnahme etwa im Hüft- oder Kniebereich. Die Anforderung kann umfassen, dass selbst bei einem Unfall, Überlastung, etc. und/oder einem möglicherweise auftretenden Versagen der Beschichtung der Grundkörper keine raue oder raspelförmige Oberfläche hat, sondern eine glatte Fläche als behelfsmäßige Artikulationsoberfläche bereitstellt.

Erfindungsgemäß wird weiterhin ein Hemiprothesen-Implantat vorgeschlagen, welches ein Gelenkersatzteil mit einer Artikulationsoberfläche zur Gleitpaarung mit einer Gelenkfläche eines natürlichen Gelenkgegenparts umfasst, wobei die Artikulationsoberfläche mit einer auf einem Untergrund aufgebrachten Beschichtung ausgebildet ist, und zumindest eine in den Untergrund hineinführende Entlüftungsbohrung und/oder eine im Untergrund verlaufende Entlüftungsnut ausgeführt ist. Entlüftungsbohrung und/oder - nut können in einem Bereich des Untergrundes unterhalb der Artikulationsoberfläche ausgebildet sein. Zusätzlich oder alternativ können Entlüftungsbohrung und/oder - nut in einem Bereich des Untergrundes außerhalb der Artikulationsoberfläche ausgebildet sein. Für Ausführungsformen derartiger Hemiprothesen-Implantate ohne Relief aber mit Entlüftungsbohrung und/oder - nut gelten, soweit anwendbar, sinngemäß die vorliegenden Beschreibungen von Ausführungsformen oder -beispielen mit Relief und mit Entlüftungsbohrung und/oder - nut.

Erfindungsgemäß wird weiterhin ein Implantatsatz oder Implantat-Set vorgeschlagen. Der Implantatsatz umfasst ein Basisimplantat zur Halterung eines Gelenkersatzteils. Der Satz umfasst weiterhin ein Totalprothesen-Implantat mit einem ersten Gelenkersatzteil und einem zweiten Gelenkersatzteil. Beide Gelenkersatzteile weisen jeweils eine Artikulationsoberfläche zur Gleitpaarung mit der jeweils anderen Artikulationsoberfläche auf. Der Satz umfasst weiterhin ein Hemiprothesen-Implantat mit einem dritten Gelenkersatzteil wie dies oben skizziert wurde oder anderweitig hierin beschrieben wird. Sowohl das erste als auch das dritte Gelenkersatzteil sind zur Halterung durch das Basisimplantat vorgesehen.

Der Implantatsatz kann ein modulares Implantatsystem bereitstellen, mittels dessen bspw. intraoperativ entschieden werden kann, ob eine Totalprothese oder eine Hemiprothese implantiert werden soll. Für eine Totalprothese ist das erste Gelenkersatzteil am Basisimplantat zu haltern oder zu verankern, für eine Hemiprothese ist das dritte Gelenkersatzteil am Basisimplantat zu haltern.

Das Basisimplantat kann bspw. einen Prothesenschaft und/oder eine anderweitige Verankerung in einem Knochen umfassen. Zusätzlich kann das Basisimplantat eine Halterungsmöglichkeit zur Halterung des ersten und/oder dritten Gelenkersatzteils bereitstellen. Es kann ein- und dieselbe Halterungsmöglichkeit für die alternative Halterung entweder des ersten oder des dritten Gelenkersatzteils vorgesehen sein. Die Halterung kann bspw. zum Aufstecken, Eindrehen, oder Verschrauben des ersten oder dritten Gelenkersatzteils am Basisimplantat vorgesehen sein.

Erfindungsgemäß wird weiterhin ein Verfahren zur Herstellung eines Hemiprothesen-Implantats vorgeschlagen. Das Verfahren umfasst das Vorsehen eines Reliefs auf einem Untergrund eines Gelenkersatzteils zur Anhaftung einer Beschichtung. Ein weiterer Schritt umfasst das Aufbringen der Beschichtung zur Ausbildung einer Artikulationsoberfläche des Gelenkersatzteils zur Gleitpaarung mit einer Gelenkfläche eines natürlichen Gelenkgegenparts.

Das Relief kann während der Herstellung des Gelenkersatzteils bspw. bei der Verarbeitung eines Werkstoffs in den Untergrund eingebracht werden. Zusätzlich oder alternativ kann zunächst ein Gelenkersatzteil ohne Relief hergestellt werden und ein Relief kann als nachgelagerter Schritt eingebracht werden.

Zum Einbringen eines Reliefs können an sich bekannte Techniken der Metallverarbeitung eingesetzt werden, wenn das Gelenkersatzteil einen Beschichtungsuntergrund aus Metall, einer Metalllegierung, etc. bereitstellt. Bspw. kann eine nachträgliche Einbringung von Reliefstrukturen wie etwa Nuten durch Fräsen erfolgen. Die Einbringung einer Aufrauhung kann mittels Strahlverfahren und/oder chemisch erfolgen. Umfasst der Beschichtungsuntergrund eine Keramik, kann ein Relief bevorzugt bereits bei der Herstellung des Gelenkersatzteils eingebracht werden, bspw. bei einem Gußvorgang, durch Modellieren, etc.

Zum Ausbilden der Beschichtung kann ein Material wie etwa ein TPE auf den mit einem Relief versehenen Untergrund aufgebracht werden, bspw. in einem Spritzgussverfahren. Das Material kann bspw. anfangs in fließfähiger Form sein, um das Relief auszufüllen, also etwa Erhebungen zu umfließen oder in Vertiefungen hineinzufließen. Danach kann das Material aushärten und kann so die Beschichtung in Form bspw. einer Kappe oder eines Mantels auf einem Grundkörper des Gelenkersatzteils ausbilden. Bei einigen Ausführungsformen kann ein Grundkörper, an dem der Untergrund ausgebildet ist, als Einlegeteil in einem Spritzgussverfahren verwendet werden.

Zusätzlich oder alternativ zu Spritzgussverfahren können andere Verfahren eingesetzt werden. Bspw. kann Beschichtungsmaterial auf den Untergrund aufgesprüht oder gesintert werden, das Gelenkersatzteil kann in ein Bad mit Beschichtungsmaterial getaucht werden, etc.. Eine Kappe, ein Mantel o.ä. zur Beschichtung kann auch zunächst separat hergestellt werden und kann dann auf dem Untergrund befestigt werden, bspw. mittels Auf- oder Einspannen, Kleben, etc..

Gemäß verschiedenen Ausführungsformen der Erfindung wird u.a. ein Implantat für Hemiprothesen bereitgestellt, bei dem ein Grundkörper aus einem steifen Material gebildet ist. Der Grundkörper aus steifem Material kann eine Kraftübertragung vermitteln, wie sie etwa in einem Gelenk auftreten kann. Der Grundkörper stellt einen Untergrund bereit, auf dem ein weniger steifes Material aufgebracht ist, um eine Artikulationsfläche auszubilden, die eine bessere Biokompatibilität haben kann als das steife Material des Grundkörpers. Diese Konfiguration bietet die Möglichkeit, als Beschichtungsmaterial ein Material zu verwenden, welches im Vergleich zu dem steifen Grundkörpermaterial zwar weniger standfest, aber besser verträglich ist. Eine Biokompatibilität kann bspw. tribologische Eigenschaften und/oder eine biologische Verträglichkeit mit natürlicherweise im Körper eines Menschen oder Tieres vorkommendem Gewebe wie Knorpel umfassen. Die Biokompatibilität kann sich so auswirken, dass ein Knorpel einer Gelenkfläche eines natürlichen Gelenks länger erhalten bleibt und/oder einen höheren Grad an Gesundheit, Intaktheit, etc. aufweist als wenn die Artikulationsfläche durch das Material des Grundkörpers gebildet würde.

Bei verschiedenen Ausführungsformen der Erfindung wird das die Artikulationsfläche ausbildende Material als Beschichtung auf den Grundkörper aufgebracht. Die Beschichtung soll möglichst sicher und dauerhaft auf dem Grundkörper befestigt sein, der die Last trägt. Hierzu ist ein Relief auf dem Beschichtungsuntergrund vorgesehen, das einer verbesserten Anhaftung der Beschichtung am Untergrund dient, und zwar im Vergleich zu einer Anhaftung an einem relieflosen Untergrund.

Das Relief kann eine Angriffs-, Eingriffs-, oder Haltestruktur ausbilden, die insbesondere für die Anhaftung eines vergleichsweise wenig steifen Materials optimiert ist, das bspw. nicht, nicht zuverlässig und/oder nicht ausreichend durch einen Press- oder Passsitz befestigt werden kann. Reliefstrukturen sollen etwa einer von der Beschichtung gebildeten Kappe insgesamt Halt bieten, so dass diese dauerhaft gegen Verschiebungen oder Verrutschen gesichert ist. Dabei soll aber lokal nicht zu stark in das wenig steife Beschichtungsmaterial eingegriffen werden, um lokale Beschädigungen zu vermeiden.

Bei einem ausreichend harten Material könnte bspw. ein einzelner Nagel, Stift oder Zahn, oder eine einzelne umlaufende Kante, Rille oder Einpassung ausreichen, um eine Befestigung zu erreichen. Gemäß Ausführungsformen der Erfindung können bei einem weniger harten oder steifen Material flächige Haltestrukturen vorgesehen werden, die aber eine lokale Überbeanspruchung des Materials vermeiden, um auch auf lange Sicht das Risiko eines Aufreißens, Durchbohrens, einer Rissbildung etc. zu minimieren und so eine dauerhafte bzw. langfristige und sichere Fügung der Beschichtung zu erreichen, ohne dass diese sich im Laufe der Zeit lockert.

Gemäß bevorzugten Ausführungsformen der Erfindung kann auch das Auftreten mechanischer Spannungen in der Beschichtung minimiert werden, die bei einem unterschiedlich ausgeprägten Anhaften der Beschichtung am Untergrund auftreten können. Gemäß verschiedenen Ausführungsformen der Erfindung können die Artikulationsfläche überdeckende Reliefs unterschiedliche Balancen zwischen flächiger Überdeckung mit Reliefstrukturen wie Zähnen oder Nuten einerseits und der lokalen Beanspruchung der Beschichtung durch diese Strukturen realisieren. Somit kann dauerhafte und sichere Fügung der Beschichtung auf dem Grundkörper für unterschiedliche Beschichtungsmaterialien, Grundkörpermaterialien, Gelenkersatztypen, Einsatzzwecke der Prothesen für Sportler, ältere Menschen, etc. optimiert werden.

Die Gefahr eines permanenten und/oder während bestimmter Gelenkbewegungen auftretenden Verrutschens oder Verschiebens der Beschichtung bzw. Kappe kann durch geeignete Ausführungsformen erfindungsgemäßer Reliefs minimiert werden, die bspw. Halt gegen tangential zum Untergrund auftretende Kräfte bieten, unabhängig davon in welche Richtungen diese Kräfte wirken. Bspw. kann ein Relief die Gefahr eines Verrutschens tangential zum Untergrund in Längsrichtung und/oder Querrichtung minimieren.

Gemäß bevorzugten Ausführungsformen der Erfindung kann ein Relief durch Strukturen wie etwa Zähne und/oder Nute gebildet werden, wobei eine einzelne Struktur so ausgebildet ist, dass sie im Vergleich zum Gesamtrelief eine geringe Kraft aufnimmt, dass also eine einzelne derartige Struktur eine Bewegung der Kappe nicht unterbinden kann. Bspw. könnten bei einem härterem Material weniger Zähne ausgebildet werden, die jedoch ausgeprägt gebildet sind, während bei einem weicherem Material mehr Zähne ausgebildet werden können, die jedoch weniger ausgeprägt sein können. Für Ausführungsformen von Reliefs mit Aufrauhung, Riffelung, etc. kann Entsprechendes gelten.

Mit Ausführungsformen der Erfindung kann ein sicherer und dauerhafter Halt der Beschichtungskappe auf dem Grundkörper zu geringen Kosten erzielt werden. Bspw. kann eine einzige Beschichtung auf dem Grundkörper genügen, so dass keine weiteren Schichten wie etwa eine oder mehrere Zwischenschichten erforderlich sind. Es genügt, ein geeignetes Relief in dem vom Grundmaterial bereitgestellten Beschichtungsuntergrund auszubilden. Dieses Relief kann durch Techniken bspw. der Werkstoffbearbeitung hergestellt werden, die an sich bekannt und kostengünstig anwendbar sind. So können etwa bekannte Spritzgusstechniken für die Beschichtung verwendet werden.

Ausführungsformen der Erfindung erweitern die Anwendungsmöglichkeiten von Hemiprothesen, bspw. indem langfristig zuverlässige Hemiprothesen mit verbesserter Verträglichkeit bereitgestellt werden. Hemiprothesen mit biokompatibler Beschichtung können mit der Erfindung bspw. auch bei starker oder langfristiger Gelenkbeanspruchung eingesetzt werden, weil die Beschichtung auch bei hoher Beanspruchung dauerhaft und zuverlässig am Grundkörper anhaftet.

Aufgrund der Möglichkeiten zur kostengünstigen Herstellung und/oder der Möglichkeit zum langfristigen Verbleib im Körper, ohne das bspw. Knorpelschäden nach vergleichsweise kurzer Zeit eine Totalprothese erforderlich machen, können Hemiprothesen gemäß der Erfindung trotz der ggf. zusätzlichen Beschichtung verstärkt als kostengünstigere Alternative zur Totalprothese zum Einsatz kommen. Hierbei werden auch Operationszeiten kürzer und allgemein die Belastungen des Patienten geringer. Hier ist es zu berücksichtigen, dass es sich bei einer späteren Versorgung der natürlichen verbliebenen Gelenkhälfte, sollte diese dennoch erforderlich werden, um eine vergleichsweise einfachere Erstimplantation handelt und nicht um eine Revision.

Gemäß Ausführungsformen der Erfindung wird ein Implantatsatz bereitgestellt, bei dem ein modulares Gelenk-Totalersatzsystem mit einem Basisimplantat im Knochen und Gelenkersatz zur Verbindung mit dem Basisimplantat durch ein Hemiprothesen-Implantat ergänzt werden kann, das einen weiteren Gelenkersatz umfasst. Beispielsweise kann ein modulares Gelenkersatz-Implantatsystem einen Satz konvexer Gelenkersatzteile (z.B. Hüftkopf, Humeruskopf, oder Femurkondyle des Knie) aus Metall oder Keramik in verschiedenen Größen für Totalersatz umfassen, wobei zu jeder Größe jeweils auch ein entsprechendes konvexes Gelenkersatzteil mit verbesserter Biokompatibilität als Hemiprothese verfügbar ist. Auf diese Weise kann ein Chirurg sich sogar noch intraoperativ flexibel für Total- oder Teilersatz entscheiden, was Vorteile für den Patienten, Behandlungskosten, etc. mit sich bringt.

Weitere Aspekte und Vorteile der Erfindung werden nachfolgend anhand der beigefügten Zeichnungen beispielhaft beschrieben. Hierbei zeigen:
- Fig. 1: in schematischer Form einen modularen Implantatsatz für Hüftgelenkersatz gemäß einem ersten Ausführungsbeispiel der Erfindung;
- Fig. 2: in schematischer, halbtransparenter Form Details des Hüft-Hemiprothesenkopfes aus Fig. 1;
- Fig. 3: in schematischer Form einen modularen Implantatsatz für Schultergelenkersatz gemäß einem zweiten Ausführungsbeispiel der Erfindung;
- Fig. 4: in schematischer, halbtransparenter Form Details des Humerus-Hemiprothesenkopfes aus Fig. 3;
- Fig. 5: in schematischer, halbtransparenter Form einen Ausschnitt einer Femur-Hemiprothesenkomponente gemäß einem dritten Ausführungsbeispiel der Erfindung;
- Fig. 6A: in Draufsicht ein Relief gemäß einem vierten Ausführungsbeispiel der Erfindung;
- Fig. 6B: in Draufsicht ein Relief gemäß einem fünften Ausführungsbeispiel der Erfindung;
- Fig. 6C: in Draufsicht ein Relief gemäß einem sechsten Ausführungsbeispiel der Erfindung;
- Fig. 7A: einen Prothesengrundkörper eines siebten Ausführungsbeispiels der Erfindung;
- Fig. 7B: eine Detailansicht des Grundkörpers der Fig. 7A mit einer erfindungsgemäßen Entlüftungsbohrung sowie Entlüftungsnut;
- Fig. 7C: eine Detailansicht des Grundkörpers der Fig. 7A mit einer erfindungsgemäßen Verankerungsnut;
- Fig. 8: einen Prothesenkopf mit Grundkörper und Beschichtung eines achten Ausführungsbeispiels der Erfindung; und
- Fig. 9: in Form eines Flussdiagramms den Ablauf eines Verfahrens zur Herstellung eines erfindungsgemäßen Hemiprothesen-Implantats.

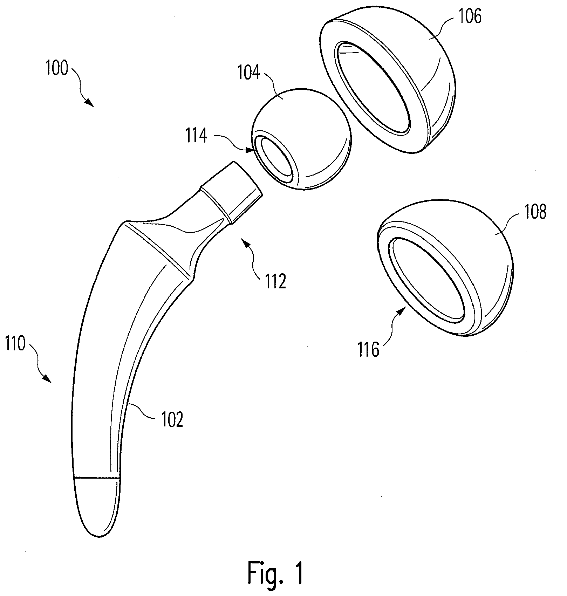

Fig. 1 zeigt in schematischer Form Komponenten eines Implantatsatzes 100 für Hüftgelenkerersatz. Der Implantatsatz 100 umfasst ein Basisimplantat, nämlich einen Hüftschaft 102, sowie als Totalprothesen-Implantate ein konvexes Totalprothesenteil, nämlich einen Totalprothesenkopf 104 und ein konkaves Totalprothesenteil, nämlich eine Totalprothesenpfanne 106, sowie als Hemiprothesen-Implantat ein konvexes Hemiprothesenteil, nämlich einen Hemiprothesenkopf 108. Der Implantatsatz 100 kann mehr als die gezeigten Komponenten umfassen. Bspw. können Basisimplantat, Prothesenköpfe und/oder Prothesenpfannen in unterschiedlichen Größen bereitgestellt werden, es können Köpfe und/oder Pfannen aus unterschiedlichem Material und/oder mit unterschiedlichen Beschichtungen bereitgestellt werden, etc..

Der Hüft- bzw. Femurschaft 102 ist zur Verankerung mit einem Ankerabschnitt 110 in einem Oberschenkelknochen vorgesehen. Bei der Implantation einer Totalprothese wird einerseits ein Oberschenkel-Gelenkkopf durch den Totalprothesenkopf 104 als Gelenkersatzteil (in Verbindung mit dem Basisimplantat 102), andererseits eine Hüftpfanne durch die Totalprothesenpfanne 106 als Gelenkersatzteil ersetzt. Für die Implantation einer Hemiprothese wird nur der Oberschenkel-Gelenkkopf durch den Hemiprothesenkopf 108 ersetzt (in Verbindung mit dem Basisimplantat 102).

Das Basisimplantat, d.h. der Hüftschaft 102 kann bspw. aus einem rostfreien Stahl, Edelstahl, einer Metalllegierung, etc. bestehen. Der Hüftschaft 102 verfügt zur Halterung von genau einem der Prothesenköpfe 104 oder 108 über einen Halterungsabschnitt 112, der im Beispiel einen Steckschaft zum Aufstecken eines der Köpfe 104 oder 108 umfasst. Jeder der Köpfe 104 und 108 verfügt über eine entsprechende Kopplung 114 bzw. 116. Im Beispiel umfasst jede Kopplung 114, 116 eine Aussparung für eine Steckverbindung mit dem Steckschaft 112 des Basisimplantats 102. Wird eine Totalprothese implantiert, wird der Prothesenkopf 104 aufgesteckt, und in der natürlichen, ggf. entsprechend vorbereiteten Hüftpfanne wird als Gegenpart zum Prothesenkopf 104 die Ersatzpfanne 106 implantiert. Eine Artikulationsoberfläche des konvexen Gelenkersatzteils 104 ist für eine Gleitpaarung mit einer Artikulationsfläche des Gelenkersatzteils 106 vorgesehen.

Wird eine Hemiprothese implantiert, wird der Prothesenkopf 108 als Gegenpart zur natürlichen Hüftpfanne aufgesteckt. Eine Artikulationsoberfläche des konvexen Gelenkersatzteils 108 ist für eine Gleitpaarung mit der natürlichen Gelenkfläche (z.B. Knorpel) der natürlichen Hüftpfanne vorgesehen.

Mit dem Implantatsatz 100 muss u.U. erst intraoperativ entschieden werden, ob eine Hemiprothese oder eine Totalprothese zu setzten ist. Ist etwa der Knorpel der natürlichen konkaven Gleitfläche des Acetabulum gesund und intakt, kann eine Hemiprothese ausreichend sein. Ist der Knorpel des Acetabulum beschädigt oder krank, kann eine Totalprothese erforderlich sein.

Wie in der Fig. 1 angedeutet, können sich die beiden Ersatzköpfe 104 und 106 in der Größe unterscheiden. Bspw. kann ein äußerere Umfang der Hemiprothese 108 eher einem äußeren Umfang des Hüftpfanneneinsatzes 106 entsprechen, d.h. der Totalprothesenkopf 104 hat wegen seines Zusammenspiels mit der Totalprothesenpfanne 106 einen kleineren Umfang als der Hemiprothesenkopf 108.

Die beiden Prothesenköpfe 104 und 108 können sich auch in den verwendeten Werkstoffen unterscheiden. Selbst wenn ein Grundkörper beider Köpfe 104, 108 aus demselben Metall oder derselben Keramik bestehen sollte, können sich die Artikulationsflächen unterscheiden. Insbesondere kann eine Artikulationsfläche des Implantats 108 im Hinblick auf eine Gleitpaarung mit einer natürlichen Gelenkfläche knorpelfreundlich beschichtet sein, während eine derartige Beschichtung für den Ersatzkopf 104 nicht erforderlich ist. Bei verschiedenen Ausführungsbeispielen kann etwa die Lauffläche des Kopfes 104 zur Gleitpaarung mit der Pfanne 106 nicht weiter beschichtet sein, oder kann etwa mit einem Material wie Polyethylen beschichtet sein, während eine Artikulationsfläche des Kopfes 108 mit PCU beschichtet sein kann. Diese Beschichtung muss dauerhaft und sicher auf dem Grundkörper des Kopfes 108 befestigt sein, wobei ein derartiger Grundkörper im Hüftgelenk auftretende Kräfte überträgt und hierzu auch die Verbindung zum Schaftsystem der Prothese bewerkstelligt.

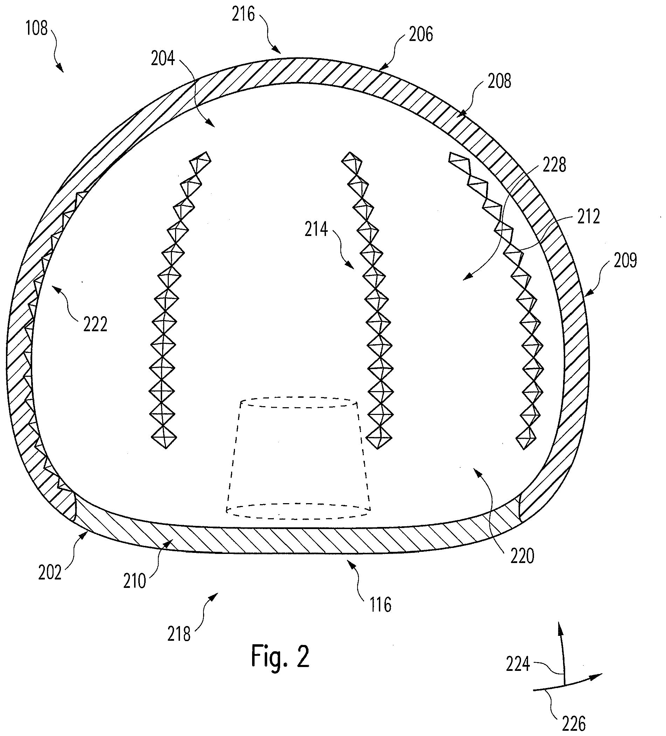

Fig. 2 ist eine semitransparente schematische Darstellung des Hemiprothesenkopfes 108 aus Fig. 1. Ein massiver Grundkörper 202 besteht etwa aus einem metallischen Material wie rostfreiem Stahl. Das Kopplungselement 116 ist in Form eines Steckkonus angedeutet.

Eine Oberfläche des Grundkörpers 202 bildet einen Untergrund 204 für die eigentliche Artikulationsfläche 206. Diese wird durch eine auf dem Untergrund 204 vorliegende Beschichtung 208 aus PCU bereitgestellt. Die Beschichtung 208 bildet im Beispiel der Fig. 2 im ausgehärteten Zustand einen Mantel bzw. eine Kappe 209 auf dem Metallkörper 202. Die Kappe 209 erstreckt sich bis zu einem Sockel 210 an der Basis des Grundkörpers 202. Der Sockel 210 kann freiliegen, nachdem sich die Artikulationsfläche 206 zur Gleitpaarung mit einer natürlichen Hüftpfanne nicht bis zum Sockel 210 erstreckt. Falls der Sockel 210 mit dem Prothesenschaft 102 (Fig. 1) in Wechselwirkung treten kann, kann ein freiliegender Sockel vorteilhaft sein, wenn die Wechselwirkungspartner aus gleichem Material oder etwa gleich hartem Material bestehen. Anders als in Fig. 1 gezeigt kann bei anderen Ausführungsbeispielen ein Beschichtungsmaterial einen Grundkörper auch vollständig abdecken, z.B. wegen allgemein besserer Biokompatibilität der Beschichtung im Vergleich zum Material eines Grundkörpers.

Der Untergrund 204 bildet eine Vielzahl erhabener Strukturen aus, nämlich Zähne 212. Die Zähne 212 können bspw. einstückig mit dem Grundkörper 202 gebildet sein, oder die Zähne 212 können separat (etwa in Form von Zahnreihen) hergestellt und bspw. derart in den Grundkörper 202 eingebracht sein, dass sie sich durch Öffnungen in der Grundfläche bzw. dem Untergrund 204 hindurch nach außen erstrecken. Die Zähne 212 sind in einer Mehrzahl von Reihen 214 angeordnet, die sich jeweils zwischen einer Spitze 216 des Kopfes und einer Basis 218 bzw. dem Sockel 210 erstrecken und in regelmäßigen Abständen um den Umfang des Kopfes 108 herum angeordnet sind. Auf diese Weise bilden die Zähne 212 ein Relief 220, welches einen großen Teil des Untergrundes 204 gleichmäßig überdeckt, wobei ein Bereich nahe der Spitze 216 und ein Bereich nahe der Basis 218 ausgenommen sein kann.

Wie etwa anhand der im Profil dargestellten Zahnreihe 222 erkennbar, greifen die Zahnreihen 214 in die Beschichtung 208 ein. Es liegt also die im Vergleich zum Grundkörper 202 weniger steife Kappe 209 nicht lediglich auf einem planen Untergrund des steiferen Grundkörpers 202 auf, sondern die Zahnreihen 214 ermöglichen eine demgegenüber verbesserte Anhaftung der PCU-Kappe 209 auf dem Stahlkörper 202. Die Zahnreihen 214 minimieren tangentiale Verschiebungen der Kappe 209 sowohl in eine Längsrichtung (Pfeil 224) als auch eine dazu senkrechte Querrichtung (Pfeil 226) und damit auch in Kombinationen derartiger Richtungen.

Das PCU-Material der Kappe 209 ist im Vergleich zum kraftübertragenden Grundkörper 202 weniger steif, d.h. das PCU-Material der Kappe 209 ist für eine dauerhafte Übertragung von im Hüftgelenk wirkenden Kräften zu wenig steif. Um dennoch eine zuverlässige Bewegungshemmung der Kappe 209 zu erreichen, sind nicht nur ein Zahn oder einige wenige Zähne vorgesehen, sondern es ist das flächige Relief 220 mit einer Vielzahl von Zähnen 212 bzw. Zahnreihen 214 ausgebildet.

Einerseits können dadurch Spannungen innerhalb der Kappe 209 minimiert werden, die bspw. zu Rißbildungen führen könnten. Andererseits muss jeder einzelne Zahn 212 oder jede einzelne Zahnreihe 214 nur einen kleinen Teil einer tangential zum Untergrund 204 wirkenden Kraft aufnehmen, so dass ein Lockern, Ausleiern, etc. des relativ weichen Materials der Kappe 209 um einen Zahn 212 oder eine Zahnreihe 214 herum minimiert werden kann und so auch Folgen wie eine vermehrte Beweglichkeit der Kappe, Rißbildungen an den Stellen der Zähne 214, etc. vermieden werden können. Zu wenige und/oder zu ausgeprägte Zähne könnten zu einem Durchbohren, Aufreißen oder anderweitiger lokaler Überbeanspruchung des Kappenmaterials führen.

Auf diese Weise kann das Relief 220 zu einer dauerhaften und zuverlässigen Befestigung der Kappe 209 auf dem Grundkörper 202 beitragen. Bei anderen Ausführungsbeispielen können bspw. neben Zahnreihen in Längsrichtung auch solche in Querrichtung, sich kreuzende Zahnreihen, etc. vorgesehen sein. Entsprechende Reliefs können bspw. abhängig vom verwendeten Beschichtungs- und/oder Grundkörpermaterial, Gelenkart bzw. erwartende Belastungen des Gelenkersatzes, zu erwartende Gelenkbewegungen und Bewegungsintensitäten, etc. ausgebildet werden.

Zusätzlich oder alternativ zu Zähnen bzw. Zahnreihen können andere reliefartige Strukturen vorliegen. Bspw. könnte das Relief 220 in flächigen Bereichen 228 zwischen den Zahnreihen 214 durch Aufrauhungen oder Riffelungen ergänzt werden, um eine Anhaftung der Kappe 209 weiter zu verbessern. Allerdings können aus nur einem Strukturelement wie bspw. Zähnen gebildete Reliefs in der Herstellung kostengünstiger sein. Statt oder zusätzlich zu Zähnen könnten bspw. Zinken in Kegel- oder Zylinderform, oder Zinnen mit zwei Kanten vorgesehen sein. Zähne könnten bspw. in verschiedenen polyedrische Formen, also etwa mit drei, vier oder mehr Kanten vorgesehen sein.

Die Form und Ausprägung einzelner Strukturelemente kann ebenfalls den Umständen der vorgesehenen Anwendung angepasst werden. Die Zähne 212 sollte einerseits groß genug sein, um eine Anhaftung der Kappe 209 gewährleisten zu können, aber nicht so groß dass die Gefahr eines Durchdringens der Beschichtung 208 besteht. Eine maximale Erhebung der Zähne 212 über ein Grundniveau des Untergrundes 204, wie es etwa durch einen zahnfreien Bereich des Untergrundes 204 definiert ist, kann daher einen Bruchteil der Dicke der Beschichtung 208 ausmachen, bspw. es können die Zähne etwa eine maximale Höhe von 70% oder weniger, 50% oder weniger, oder 30% oder weniger der Dicke der Beschichtung 208 haben.

Es könnten Zähne unterschiedlicher Größe und/oder Ausprägung in ein- und demselben Relief verwendet werden. Bspw. könnten Zähne nahe der Spitze und/oder der Basis eines Gelenkkopfersatzes kleiner ausfallen als in einem mittleren Bereich.

Andere Ausführungsbeispiele von Implantatsätzen für bspw. Hüftprothesen sind nicht modular ausgeführt, sondern enthalten eine einstückig als Femurschaft mit Kopf ausgeführte Totalprothese, bspw. einen Monoblock-Hüftschaft mit Gelenkkugel, und dazu mindestens einen Hüftpfannenersatz, sowie eine einstückig als Femurschaft mit Kopf ausgeführte Hemiprothese, bspw. ebenfalls einen Monoblock-Hüftschaft mit Gelenkkugel. Ggf. liegen einzelne oder alle dieser Komponenten in unterschiedlichen Größen vor. Die Eigenschaften der Hemiprothese im Kopfbereich können denen entsprechen die oben für den modularen Systemkopf 108 beschrieben wurden, außer dass die Kopplung 218 entfällt.

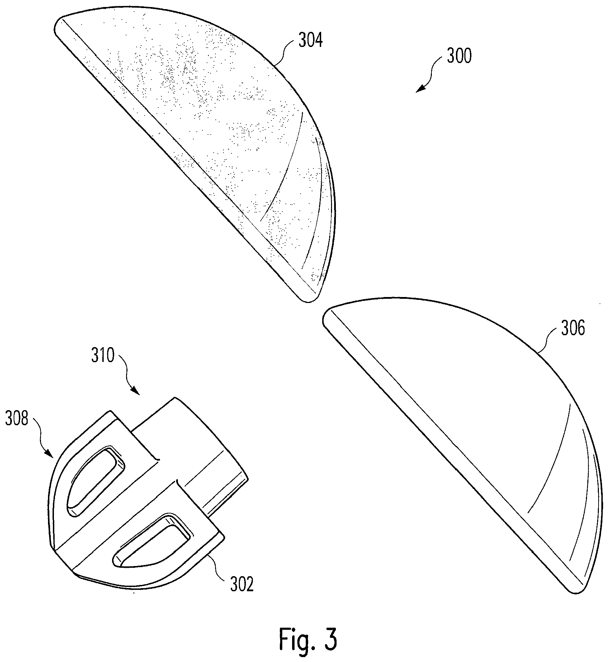

Fig. 3 zeigt in schematischer Form Komponenten eines Implantatsatzes 300 für Schultergelenkersatz. Der Implantatsatz 300 umfasst ein Basisimplantat, nämlich einen Humeruskurzschaft 302, sowie als Totalprothesen-Implantat ein konvexes Totalprothesenteil, nämlich einen Totalprothesenkopf 304, zu dem ein nicht dargestelltes konkaves Totalprothesenteil gehört, nämlich eine Totalprothesenschulterpfanne als Ersatz von Schulterpfanne / Glenoid. Der Implantatsatz 300 umfasst weiterhin als Hemiprothesen-Implantat ein konvexes Hemiprothesenteil, nämlich einen Hemiprothesenhumeruskopf 306. Der Implantatsatz 300 kann mehr als die gezeigten Komponenten umfassen, bspw. können Schaft, Prothesenköpfe und/oder Prothesenpfannen in unterschiedlichen Größen, aus unterschiedlichen Materialien, etc. bereitgestellt werden.

Die Kurzschaftprothese 302 ist zur Verankerung im Oberarmknochen mittels eines Ankerbereichs 308 mit Knochenfensterung vorgesehen. Bei der Implantation einer Totalprothese wird unter anderem ein Oberarm-Gelenkkopf durch den Totalprothesenkopf 304 ersetzt. Für die Implantation einer Hemiprothese wird der Oberarm-Gelenkkopf durch den Hemiprothesenkopf 306 ersetzt.

Der Humerusschaft 302 kann bspw. aus einem Metall wie Titan, einer Titanlegierung, etc. bestehen. Ggf. kann eine Beschichtung vorgesehen sein, etwa am Anker 308, die ein sicheres Verankern durch Einwachsen im Knochen fördert. Der Schaft 302 verfügt zur Halterung von genau einem der Prothesenköpfe 304 oder 306 über einen Halterungsabschnitt 310, der bspw. einen Steckschaft zum Aufstecken eines der Köpfe 304 oder 306 umfasst. Wird eine Totalprothese implantiert, wird der Prothesenkopf 304 auf den Schaft aufgesteckt und ein Glenoid-Gelenkersatzteil als Gegenpart implantiert. Wird eine Hemiprothese implantiert, wird der Prothesenkopf 306 als Gegenpart zum natürlichen Glenoid aufgesteckt.

Mit dem Implantatsatz 100 muss u.U. erst intraoperativ entschieden werden, ob eine Hemiprothese oder eine Totalprothese zu setzten ist. Ist etwa der Knorpel der natürlichen, konkaven Gleitfläche des Glenoid gesund und intakt, kann eine Hemiprothese ausreichend sein. Ist der Glenoidknorpel beschädigt oder krank, kann eine Totalprothese erforderlich sein.

Dementsprechend ist eine Artikulationsoberfläche des Gelenkersatzteils 304 an eine Gleitpaarung mit einer Artikulationsfläche des Glenoid-Gelenkersatzteils angepasst. Eine Artikulationsoberfläche des konvexen Gelenkersatzteils 306 ist für eine Gleitpaarung mit der natürlichen Gelenkfläche (z.B. Knorpel) des natürlichen Glenoids vorgesehen.

Die beiden Prothesenköpfe 304, 306 können sich in den Werkstoffen bzw. Materialien, im Vorkommen und der Zusammensetzung von Beschichtungen, etc. unterscheiden. Selbst wenn die Grundkörper beider Köpfe 304 und 306 aus dem gleichen Grundstoff wie einem Metall oder einer Keramik bestehen sollten, können die Artikulationsflächen von unterschiedlichen Materialien gebildet sein. In einem Ausführungsbeispiel ist die Lauffläche des Implantats 304 nicht weiter beschichtet, während die Artikulationsfläche des Kopfes 306 von einer Beschichtung gebildet ist. Die Beschichtung des Kopfes 306 kann etwa aus PCU bestehen und sollte untrennbar auf dem Grundkörper des Kopfes 306 befestigt sein, der die Last trägt und die Verbindung zum Schaftsystem 302 der Prothese bewerkstelligt.

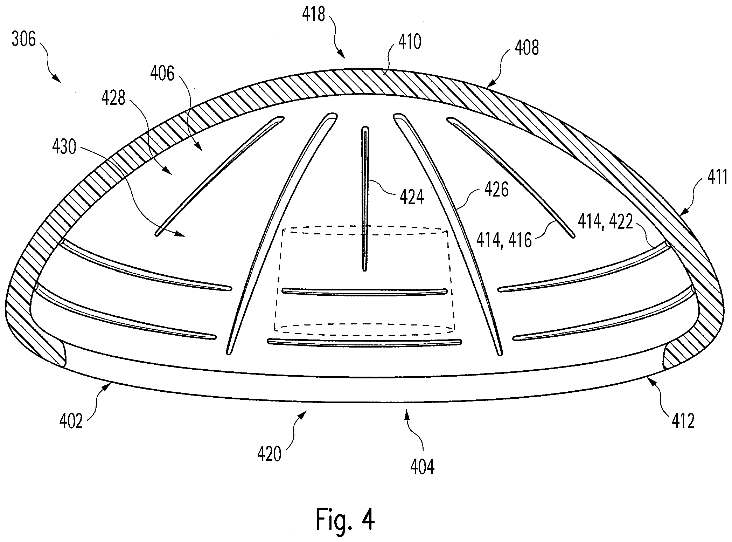

Fig. 4 ist eine semitransparente schematische Darstellung des Hemiprothesenkopfes 306 aus Fig. 3. Ein massiver Grundkörper 402 besteht etwa aus einem keramischen Material. Ein Kopplungselement 404 in Form eines Steckkonus dient zur Kopplung mit dem Schaft 302.

Eine Oberfläche des Grundkörpers 402 bildet einen Untergrund 406 für die eigentliche Artikulationsfläche 408. Diese wird durch eine auf dem Untergrund 406 vorliegende Beschichtung 410 aus PCU bereitgestellt. Die Beschichtung 410 bildet im Beispiel der Fig. 4 im ausgehärteten Zustand einen Mantel bzw. eine Kappe 411 auf dem Keramikkörper 402. Die Kappe 411 kann eine Oberseite bzw. äußere Oberfläche des Körpers 402 vollständig abdecken und kann auf eine Unterseite des Kopfes 306 untergreifen, an welcher sich die Kopplung 404 befindet. Insbesondere kann sich die Kappe 411 bis zu einem zurückversetzten Sockel 412 erstrecken.

Der Untergrund 406 bildet eine Vielzahl Nute 414 aus (Verankerungsnute). Es sind einerseits Längsnute 416 vorgesehen, die sich zwischen einer Spitze 418 des Kopfes und einer Basis 420 bzw. dem Sockel 412 erstrecken und in regelmäßigen Abständen um den Umfang des Kopfes 306 herum angeordnet sind. Weiterhin sind Quernute 422 vorgesehen, die entlang von Umfangslinien etwa in einen Bereich nahe der Basis 420 verlaufen.

Es sind längere Längsnuten 424 und kürzere Längsnuten 426 vorgesehen. Die Quernute 422 verlaufen nicht um den gesamten Kopf 306 herum, sondern sind unterbrochen bzw. erstrecken sich zwischen den Längsnuten 426. Auf diese Weise bilden die Nute 414 ein Relief 428 welches den Untergrund 406 überdeckt. Das Relief 428 ist lokal unterschiedlich ausgeprägt, nachdem die Quernute 414 bevorzugt nahe der Basis 420 vorliegen.

Die von der Beschichtung 410 gebildete Kappe 411 liegt nicht nur auf dem Untergrund 406 auf, sondern greift in die Nute 414 des Grundkörpers 402 ein. Es liegt also die im Vergleich zum Grundkörper 402 weniger steife Kappe 411 nicht lediglich auf einem lokal planen Untergrund des steiferen Grundkörpers 402 auf, sondern die Nute 414 ermöglichen eine demgegenüber verbesserte Anhaftung der PCU-Kappe 411 auf dem Keramikkörper 402. Die Längsnute 416 minimieren Querverschiebungen der Kappe 411, und die Quernute 422 minimieren Längsverschiebungen der Kappe 411 und verbessern auf diese Weise den Halt der Kappe 411.

Das PCU-Material der Kappe 411 ist im Vergleich zum kraftübertragenden Grundkörper 402 weniger steif bzw. weicher und flexibler. Um dennoch eine zuverlässige Bewegungshemmung der Kappe 411 zu erreichen, ist es vorteilhaft, nicht nur eine einzige Längsnut und/oder eine einzige Quernut (oder eine wenige punktuelle Vertiefungen) im Untergrund 406 vorzusehen, sondern ein flächiges Relief wie in Fig. 4 beispielhaft mit dem Relief 428 angedeutet.

Einerseits können durch das Relief 428 Spannungen innerhalb der Kappe 411 minimiert werden, die aufgrund von Bewegungen von Teilen der Kappe 411 gegeneinander zu Rißbildungen führen könnten. Andererseits muss jede einzelne Rippe (bzw. Kante, Kamm) der Beschichtung 410, die in eine Nut 414 eingreift, nur einen kleinen Teil einer tangential zum Untergrund 406 wirkenden Kraft aufnehmen, so dass ein Lockern oder Ausleiern des Materials der Kappe 411 im Bereich der Nute minimiert werden kann. Zu wenige und/oder zu ausgeprägte Nute könnten zu lokalen Unterschieden in der Steifigkeit der Beschichtung 410 und in der Folge zu Rißbildungen oder anderen lokalen Überbeanspruchungen der Kappe führen.

Auf diese Weise kann das Relief 428 zu einer dauerhaften und zuverlässigen Befestigung der Kappe 411 auf dem Grundkörper 402 beitragen. Zusätzlich oder alternativ zu Nuten können auch andere reliefartige Strukturen vorliegen. Bspw. könnte das Relief 428 durch aufgeraute flächige Bereiche 430 zwischen den Nuten 414 ergänzt werden, um eine Anhaftung der Kappe 411 weiter zu verbessern.

Die Nute 414 sollten tief genug sein, um eine Anhaftung der Kappe 411 gewährleisten zu können. Eine maximale Tiefe der Nute unter ein Grundniveau des Untergrundes 406 wie es etwa durch einen nutenfreien Bereich des Untergrundes 406 lokal definiert ist, kann daher einen Bruchteil der Dicke der Beschichtung 410 ausmachen. Bspw. können die Nute etwa eine maximale Tiefe von 70% oder weniger, 50% oder weniger, oder 30% oder weniger als die Dicke der Beschichtung 410 haben.

Ein Profil der Nute kann je nach Material des Untergrundes 406, der Beschichtung 410, und/oder anderen Umständen des Einzelfalles ausgeführt werden. Bspw. können die Nute in Dreiecksform, rechteckförmig, trogförmig, und/oder wieder anders vorgesehen sein. Tiefe, Profil und/oder Anzahl der Nute können auch in Zusammenhang stehen. Bspw. kann im Vergleich zu einer gegebenen Konfiguration eine höhere Anzahl an Nuten vorgesehen werden, die aber flacher sind, um eine vorgegebene Anhaftungswirkung zu erreichen. Werden sehr viele sich kreuzende Nute angelegt, gelangt man zu einem Relief mit Riffelung, wie es beispielhaft weiter unten diskutiert wird.

Bei einigen Ausführungsbeispielen könnten mehr Quernute vorgesehen sein als in Fig. 4 angedeutet sind; die zusätzlichen Quernute könnten sich etwa näher an einer Spitze des Grundkörpers erstrecken. Bei anderen Ausführungsbeispielen könnten mehr Längsnute vorgesehen sein. Die Quernute 414 in Fig. 4 sind an den Stellen der Längsnute 426 mit Unterbrechungen versehen. Unterbrechungen könnten auch in den Längsnuten vorgesehen sein. Statt nur Längs- und/oder Quernute vorzusehen, könnten zusätzlich oder alternativ auch Nute mit anderen Orientierungen vorgesehen sein, bspw. schräge Nute. Nute könnten sich auch ohne Unterbrechungen kreuzen. Ein Relief mit kreuzenden Nuten wird unten beschrieben.

Andere Ausführungsbeispiele eines Implantatsatzes für Schulterprothesen sind nicht modular ausgeführt, sondern enthalten eine einstückig als Humerusschaft mit Kopf ausgeführte Totalprothese, bspw. ein Monoblock-Schulterschaftsystem mit Humeruskugel, und dazu mindestens einen Glenoidersatz, sowie eine einstückig als Humerusschaft mit Kopf ausgeführte Hemiprothese, bspw. ebenfalls ein Monoblock-Schulterschaftsystem mit Humeruskugel. Ggf. liegen einzelne oder alle dieser Komponenten in unterschiedlichen Größen vor. Die Eigenschaften der Hemiprothese am Kopfbereich können denen entsprechen die oben für den modularen Systemkopf 306 beschrieben wurden, außer dass die Kopplung 404 entfällt.

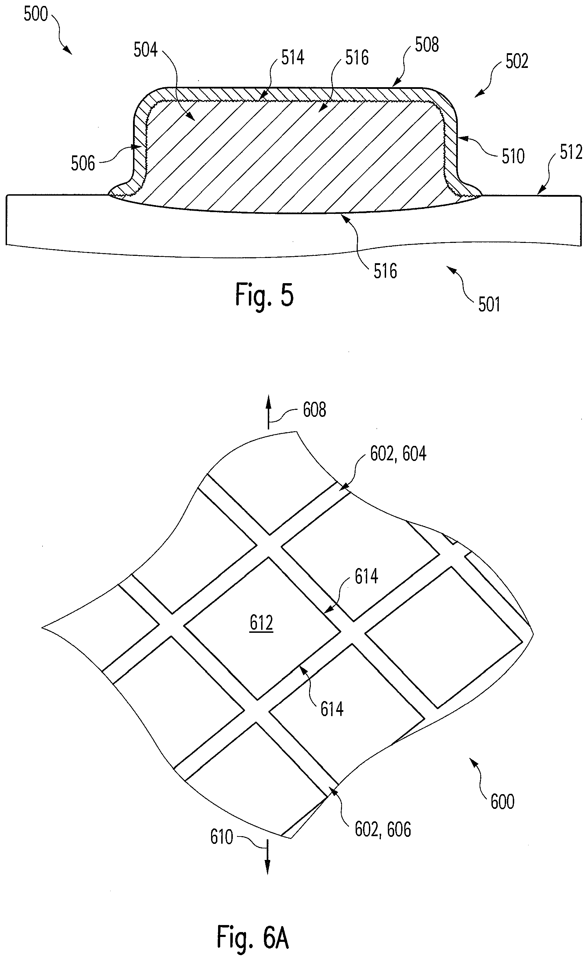

Fig. 5 zeigt in schematischer semitransparenter Teilansicht ein Implantat 500 zur Verwendung als Femur- bzw. Condylus-Gelenkersatz in Form einer Hemiprothese. Das Implantat 500 kann bspw. Teil eines Implantatsatzes für Knieprothesen sein, wobei der Satz neben der Hemiprothese 500 z.B. auch eine Totalprothese umfassen könnte.

Das Implantat 500 umfasst einen Grundkörper 501 mit einem Condylusersatz bzw. einer Gelenkrolle 502. Die Rolle 502 kann massiv sein und kann bspw. aus rostfreiem Stahl ausgeführt sein. Grundkörper 501 und Gelenkrolle 502 werden nachfolgend gelegentlich gleichgesetzt. Eine Oberfläche der Rolle 502 bildet einen Untergrund 504 für eine knorpelfreundliche Beschichtung 506, die bspw. PCU umfassen kann. Die Beschichtung 506 bildet eine Artikulationsfläche 508 zur Artikulation mit einer Gelenkfläche eines natürlichen Oberschenkelknochens aus.

Die Beschichtung 506 bildet einen Mantel bzw. eine Kappe 510 auf der Rolle 502 aus, wobei die Kappe 510 eine äußere Oberfläche der Rolle 502 vollständig abdeckt. Bei anderen Ausführungsformen kann die Beschichtung 506 auch weitere Oberflächenbereiche 512 des Grundkörpers 501 abdecken.

Der Untergrund 504 wird durch eine aufgeraute Oberfläche gebildet, die in Fig. 5 durch eine unregelmäßige Konturlinie 514 und Schraffur 516 angedeutet ist und im Beispiel die gesamte Rolle 502 überdeckt. Somit liegt die Kappe 510 nicht auf einem lokal planen Untergrund auf, sondern steht im Angriff mit der aufgerauten Oberfläche des Körpers 502, so dass auf diese Weise eine verbesserte Anhaftung der Kappe 510 auf der Rolle 502 gewährleistet ist.

Der raue Untergrund 504 minimiert Verschiebungen der Kappe 510 aufgrund von in Bezug auf den Untergrund 504 tangentialen Kraftwirkungen. Die Aufrauhung 514, 516 ist ein weiteres Beispiel 518 für ein flächiges Relief, das geeignet ist, um in unterschiedlichen Bereichen der Artikulationsfläche 508 tangentiale Kräfte lokal aufzunehmen und weiterzuleiten und so einerseits unerwünschten Bewegungen oder Verschiebungen der Kappe 510 und andererseits das Auftreten von Rissen oder anderen Schäden an der Beschichtung 506 zu minieren.

Anders als in Fig. 5 angedeutet könnten auch nur Teilbereiche einer Rolle, eines Kopfes oder einer sonstigen Komponente zur Gleitpaarung aufgeraut sein. Die Vertiefungen und Erhebungen des Reliefs 518 bzw. der Aufrauhung 514, 516 sollten ausgeprägt genug sein, um eine verbesserte Anhaftung der Kappe 510 im Vergleich zu einem planen Untergrund gewährleisten zu können. Die maximalen Erhöhungen bzw. Vertiefungen können sich in der Größenordnung von Bruchteilen einer Dicke der Beschichtung bewegen und können bspw. 70% oder weniger, 50% oder weniger, oder 30% oder weniger einer Dicke der Beschichtung 506 betragen.

Fig. 6A zeigt schematisch eine Draufsicht auf einen Ausschnitt eines Reliefs 600 wie es in bzw. an oder auf einem Untergrund eines Hemiprothesen-Implantats ausgeprägt sein könnte, um eine Anhaftung für eine Beschichtung zur Ausbildung einer Artikulationsfläche zu optimieren. Im Ausführungsbeispiel der Fig. 4 umfasst das Relief 428 eine Mehrzahl von kreuzungsfrei ausgeführten Nuten 414. Im Ausführungsbeispiel der Fig. 6A umfasst das Relief 600 hingegen eine Mehrzahl von sich kreuzenden Nuten 602. Bei parallelen Nuten 604 könnte es sich bspw. um Längsnute handeln und bei parallelen Nuten 606 könnte es sich um Quernute handeln. Die Nute 604, 606 können aber auch in andere Richtungen verlaufen, bspw. in diagonale Richtungen bezogen etwa auf eine Spitze oder eine Basis eines bspw. konvexen Gelenkersatzteils, wobei die Richtung zur Spitze bzw. Basis durch Pfeile 608 bzw. 610 angedeutet ist.

Für die Ausbildung der Nute 602 gilt entsprechendes wie für die Nute 412 der Fig. 4 diskutiert. Durch die Nute 602 werden erhabene Bereiche 612 definiert, deren Kanten 614 im Falle von tangentialen Kraftwirkungen, die an einer anlagernden Beschichtung angreifen, zu einer Bewegungshemmung und somit zu einer verbesserten Anhaftung der Beschichtung auf dem Untergrund führen. Wie das Beispiel der Fig. 6A verdeutlicht, kann das Relief 600 einerseits als eine durch Nute 602 gegebene Musterung beschrieben werden, wobei die Nute 602 Vertiefungen gegenüber einer Grundfläche bzw. eines gedacht planen Untergrundes sind. Jedoch ist diese Beschreibung willkürlich, denn das Relief 600 kann andererseits auch als Muster erhabener Bereiche 612 beschrieben werden. Welche Beschreibungsform gewählt wird, kann etwa von der Herstellung abhängig gemacht werden, bspw. wenn das Relief 600 durch Einbringung von Nuten 602 erzeugt wird. Andere Herangehensweisen sind aber ebenfalls denkbar.

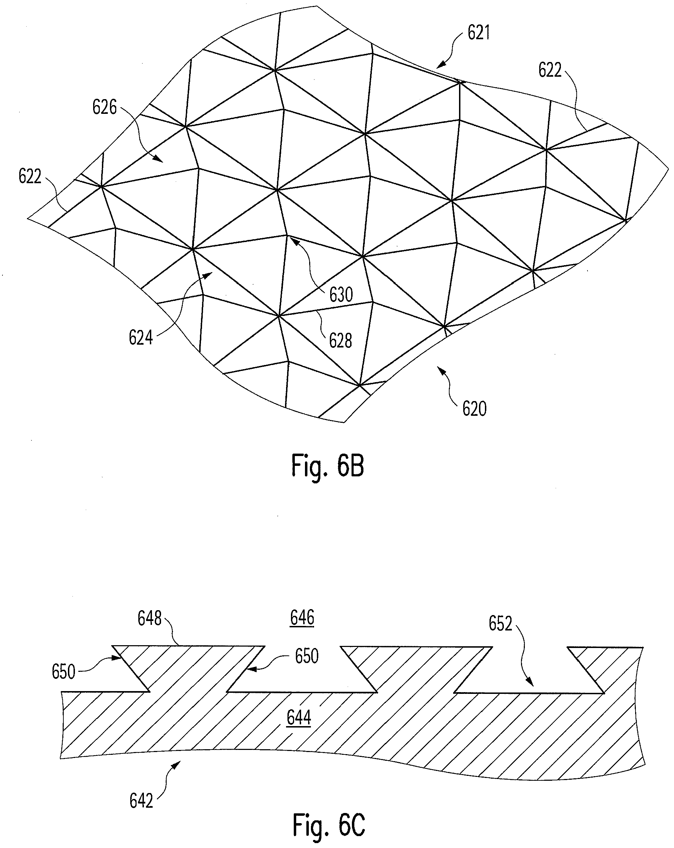

Fig. 6B zeigt schematisch einen Ausschnitt in Draufsicht aus einem weiteren Ausführungsbeispiel eines Reliefs 620. Dieses umfasst eine Riffelung 621 zur Verbesserung einer Anhaftung einer Beschichtung. Die Riffelung 621 umfasst eine Vielzahl von lediglich durch Linien angedeuteten Nuten, Rinnen oder Rillen 622, die eine Vielzahl von erhabenen Bereichen 624 definieren. Jeder dieser Bereiche bildet einen Zahn 626 aus. Jeder Zahn hat vier Kanten oder Grate 628 und eine Spitze 630. Andere Gestaltungen, bspw. andere polyedrische Formen mit weniger oder mehr Kanten, sind ebenfalls denkbar.

Fig. 6C zeigt einen schematischen Querschnitt durch einen Untergrund 642, der von einer Oberfläche eines Grundkörpers 644 gebildet wird, und in dem zur verbesserten Anhaftung einer Beschichtung 646 eine Vielzahl von Erhebungen 648 ausgebildet sind. Die Erhebungen 648 weisen in einem der Beschichtung 646 zugewandten Bereich jeweils zwei hakenartige Strukturen 650 auf. Die Haken 650 können wie gezeigt beidseitig oder auch nur einseitig ausgebildet sein.

Gemäß einer komplementären Beschreibung der Konfiguration der Fig. 6C sind Nute, Rinnen oder Vertiefungen 652 in den Untergrund 642 eingebracht, wobei die Nute 652 überhängende Kanten aufweisen. Strukturen wie die in Fig. 6C gezeigten könnten in einen metallischen Grundkörper bspw. durch fräsende Metallbearbeitung oder in einen keramischen Grundkörper mittels Gußkeramik eingebracht werden.

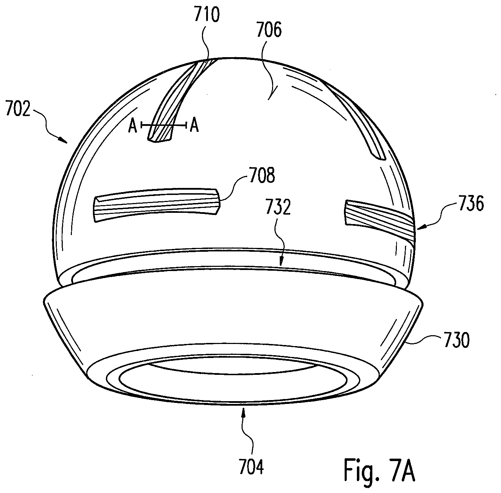

Fig. 7A zeigt ein Ausführungsbeispiel eines Grundkörpers 702 für einen Hemiprothesenkopf, wobei hier Beschichtung bzw. Kappe oder Mantel weggelassen sind. Für einen Hemiprothesenkopf, für den der Grundkörper 702 verwendet werden könnte, gilt sinngemäß das was in Bezug auf den Prothesenkopf 108 der Fig. 1 und 2 diskutiert wurde.

Der Grundkörper 702 kann aus einem metallischen Material wie bspw. Stahl ausgeführt sein. Ein Kopplungselement ist in Form eines Steckkonus 704 angedeutet. Eine Oberfläche des Grundkörpers 702 bildet einen Untergrund 706 für eine aufzubringende Beschichtung. Der Untergrund 706 bildet eine Vielzahl Nute 708 aus. Falls nichts anderes gesagt ist, gilt für diese Nute 708 vergleichbares wie es etwa für die Nute 414 des Ausführungsbeispiels der Fig. 4 diskutiert wurde.

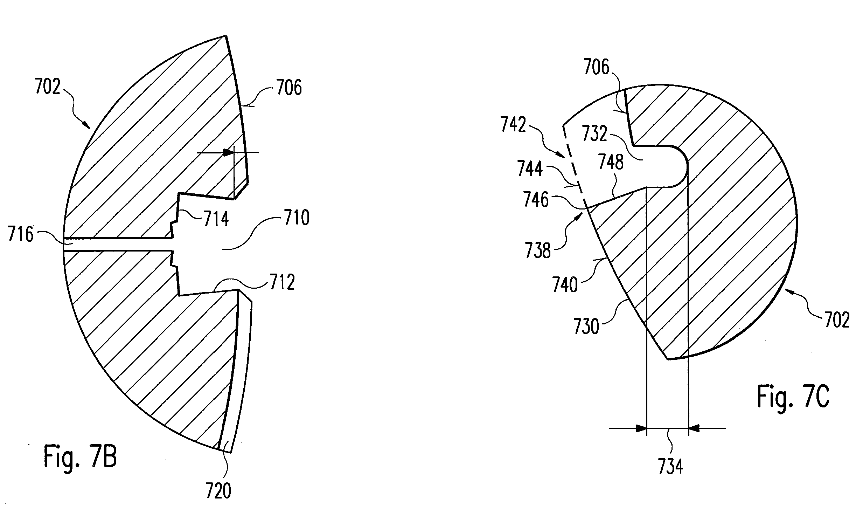

Fig. 7B zeigt ein Querschnittsprofil durch den Grundkörper 702 mit der Nut 710 entlang der Linie A-A in der Fig. 7A. Die Nut 710 ist mit seitlichen Wandungen 712 und einem Boden 714 in den Grundkörper 702 eingebracht. Die Seitenwände 712 können als Hinterschneidungen, d.h. mit überhängenden Kanten ausgebildet sein, wie anhand der Fig. 6C beschrieben wurde. In den Boden 714 der Nut 712 ist eine Bohrung 716 eingebracht, die sich bis durchgehend bis zu einem Innenbereich des Grundkörpers 702 erstrecken kann. Bspw. kann die Bohrung 716 im Konus 704 münden, vgl. Fig. 7A.

Im Zuge der Fertigung der Hemiprothese 700 wird der Untergrund 706 mit einem Kunststoff beschichtet, bspw. in einem Spritzgußverfahren. Hierbei kann der Grundkörper 702 als metallisches Einlegeteil verwendet werden. Beim Überspritzen des Untergrundes 706 mit dem Kunststoff dient die Bohrung 716 der Entlüftung. Dies kann bspw. die Haftung der resultierenden Beschichtung bzw. Kappe verbessern.

Wie in Fig. 7B angedeutet, kann im Allgemeinen eine von einer Entlüftungsbohrung 716 eingenommene Fläche des Untergrundes 706 merklich kleiner sein als eine von einer Vertiefung wie etwa der Nut 710 bzw. dessen Boden 714 eingenommene Fläche. Entlüftungsbohrungen, -rinnen, oder - öffnungen können etwa weniger als 50%, weniger als 30%, weniger als 20%, oder weniger als 10% der Bodenfläche einer Nut oder anderen Vertiefung ausmachen.

Die Entlüftungsbohrung 716 führt senkrecht vom Untergrund 706 bzw. dem Boden 714 der Nut 710 weg in das Innere des Grundkörpers 702, d.h. in einem Winkel von 90° zur lokalen Oberfläche 706, 714 des Grundkörpers 702. Bei anderen Ausführungsbeispielen kann die Bohrung auch schräg verlaufen, d.h. unter einem anderen Winkel als 90°, also z.B. unter einem Winkel von 80° oder 70°. Dies kann z.B. vorteilhaft sein, damit eine Bohrung einen inneren Hohlraum des Grundkörpers erreichen kann.

In Fig. 7B ist weiterhin eine Entlüftungsnut 720 angedeutet, die in den Untergrund 702 eingebracht ist, und zwar von der Nut 710 ausgehend und von dieser wegführend. Auch die Nut 720 dient der Entlüftung und damit erzielbaren verbesserten Anhaftung einer aufgebrachten Beschichtung auf den Untergrund 706 bzw. am Grundkörper 702. Die Ausführungen zur Entlüftungsbohrung 716 gelten, soweit anwendbar, sinngemäß auch für die Entlüftungsnut 720.

Entlüftungsnute können senkrecht, d.h. in einem Winkel von 90° von einer Nut wie der Nut 710 wegführen, oder können in einem anderen Winkel wegführen, bspw. 75° oder 45°. Entlüftungsnute können kurz sein, so dass eine Länge der Entlüftungsnut bspw. kleiner oder gleich der Breite oder Tiefe der zu entlüftenden Vertiefung ist, bspw. einer Nut. Entlüftungsnute können aber auch lang sein und sich um ein Mehrfaches der Breite einer zu entlüftenden Vertiefung erstrecken. Bei bestimmten Ausführungsbeispielen kann eine Entlüftungsnut mehrere zu entlüftende Strukturen wie etwa mehrere Nute wie die Nute 708 in Fig. 7A miteinander verbinden.

Es können mehrere Entlüftungsbohrungen und/oder -nute pro zu entlüftender Struktur vorgesehen sein. Bpsw. können auf jeder Seite der Nut 710 in Fig. 7A 3 Entlüftungsnute, oder 10 Entlüftungsnute vorgesehen sein.

Entlüftungsbohrungen wie die Bohrung 716 können in einer Vertiefung angelegt sein, also bspw. einer Nut wie Nut 710 in Fig. 7B, in Nuten 602 in Fig. 6A, in Rillen 622 wie in Fig. 6B, etc. Ebenso können Entlüftungsnute von derartigen Vertiefungen wegführen.

In der Fig. 7A ist gezeigt, dass der Beschichtungsuntergrund 706 angrenzend an einen Sockel 730 des Grundkörpers 702 mit einer umlaufenden Verankerungsnut 732 versehen ist.

Fig. 7C zeigt ein weiteres Querschnittsprofil durch den Grundkörper 702, wobei das Profil den Sockel 730 und die Verankerungsnut 732 schneidet. Die Nut 732 kann in Abhängigkeit vom verwendeten Beschichtungsmaterial vorgesehen werden, um eine Verankerung der Beschichtung bzw. des von der Beschichtung gebildeten Mantels auf dem Grundkörper 702 zu optimieren. Eine Tiefe 734 der Nut 732 und/oder weitere Auslegungsparameter können je nach Beschichtungsmaterial, Verwendungszweck, d.h. Art der Prothese und übertragene Kräfte, etc., festgelegt werden.

Die Nut 732 ist in Fig. 7A umlaufend gezeigt, jedoch sind Abwandlungen möglich; so kann bspw. eine Verankerungsnut nur stück- bzw. segmentweise ausgebildet sein. Statt einer ganz oder teilweise umlaufenden Verankerungsnut können auch mehrere (Quer-)Verankerungsnute parallel zueinander vorgesehen sein, wobei der Übergang zu Quernuten 736 fließend verlaufen kann.

Prinzipiell ist es denkbar, dass eine Verankerungsnut in einem Abstand zu einem Sockelbereich des Grundkörpers vorgesehen ist; gegenwärtig wird aber für den Fall, dass eine Verankerungsnut für zweckmäßig angesehen wird, die in den Fig. 7A und 7C gezeigte Konfiguration bevorzugt, bei der die Verankerungsnut 732 unmittelbar am Sockel 730 vorgesehen ist. Hierdurch wird am Übergang 738 ein Fügespalt minimiert, der zwischen Grundkörper 702 bzw. Außenfläche 740 des Sockels 730 einerseits und einer aufgebrachten Beschichtung andererseits auftreten kann, wobei die Beschichtung nach dem Aufbringen eine durch die gestrichelte Linie angedeutete Kappe 742 ausbildet. Die Kappe 742 kann bspw. durch ein spritzgegossenes PCU-Material gebildet werden.

Um einen Fügespalt am Übergang 738 von der PCU-Gleitfläche 744 der Kappe 742 auf die metallische Sockelaußenfläche 740 Spalt zu minimieren, kann die äußere Kante oder Ecke 746 der Sockelhinterschneidung 748 des Sockels 712 scharfkantig ausgeführt sein, und die Sockelkante 748 kann ansonsten gerade und strukturfrei in Richtung auf den Untergrund 706 bzw. die Nut 732 verlaufen. Auf diese Weise kann eine bündige Anlagerung von bspw. spritzgegossenem Material an die Sockelkante 748 erleichtert werden. Würde die Kante 746 abgerundet ausgeführt, kann es sein dass das verwendete Spritzgussmaterial nicht exakt bis an die äußere Sockelkante benetzt. In derartigen Fällen könnte sich am Übergang zwischen Kunststoff- und Metalloberfläche bspw. eine Eintiefung oder Mulde ausbilden.

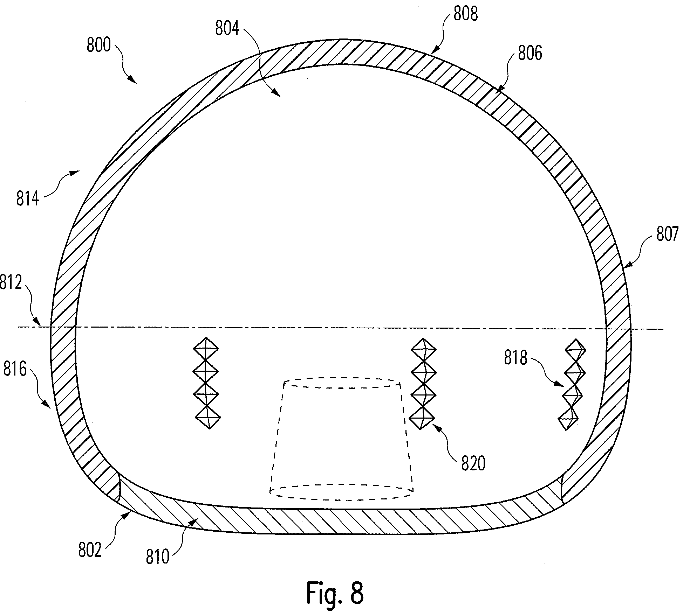

Fig. 8 zeigt eine Darstellung eines Hemiprothesenkopfes 800, bei dem es sich um eine Variation des Prothesenkopfes 108 der Fig. 2 handeln kann; solche Aspekte, bei denen sich die beiden Köpfe 108 und 800 gleichen können, werden nachfolgend nicht erneut explizit beschrieben.

Ein Grundkörper 802 bietet einen Untergrund 804 für eine Beschichtung 806 bzw. Kappe 807, auf der bzw. durch welche die Artikulationsfläche 808 gebildet ist. Genauer gesagt überdeckt die Kappe 807 den Grundkörper 802, wobei sich die Kappe 807 bis an einen Sockel 810 des Kopfes 800 erstreckt. Ohne Sockel 810 hat der Kopf 802 eine angenähert sphärische Form, so dass auch eine Oberfläche der Kappe 807 Kugelform annimmt.

In Fig. 8 ist ein Äquator 812 eingezeichnet, wobei der Begriff 'Äquator' sich auf die angenäherte Kugelform der Kappe 807 bezieht, wie dies dem Fachmann bekannt ist. Die Oberfläche der Kappe 807 in einem Bereich 814 oberhalb des Äquators 812 stellt die eigentliche Artikulationsoberfläche 808 dar, d.h. bei normalen Gelenkbewegungen stellt der Bereich 814 die artikulierende Zone bzw. den Artikulationsbereich in Wechselwirkung mit dem (natürlichen) Gelenkgegenpart bereit. Ein Bereich 816 unterhalb des Äquators 812 gehört im normalen Bewegungsumfang nicht zur artikulierenden Zone 808.

Der Untergrund 804 bildet eine Vielzahl Zähne 818 aus, wie dies im Detail für das Ausführungsbeispiel der Fig. 2 mit Zähnen 212 beschrieben wurde. Die Zähne 818 sind auch im Beispiel der Fig. 8 in einer Mehrzahl Zahnreihen 820 angeordnet. Im Unterschied zu dem Ausführungsbeispiel der Fig. 2 sind allerdings die Zahnreihen 820 nur außerhalb der normalen Artikulationszone 814 vorgesehen, d.h. in dem an den Sockel 810 angrenzenden Bereich 816, während im Bereich 814, d.h. unterhalb der Artikulationszone 808, der Untergrund 804 frei von Zähnen oder anderen Reliefstrukturen ist, d.h. der Untergrund 804 ist unterhalb der Artikulationsoberfläche 808 plan.

Eine mit den Zahnreihen 820 erzielte Haft- bzw. Verankerungswirkung der Kappe 807 auf dem Grundkörper 802 kann ausreichend sein, so dass auf ein Relief unter der Artikulationsoberfläche verzichtet werden kann. Ist umgekehrt ein Verzicht auf ein Relief unterhalb der Artikulationszone 808 gefordert, etwa aus Sicherheitsgründen, kann dennoch eine ausreichende Haftwirkung erzielt werden, wie dies am Bsp. der Fig. 8 gezeigt wird. In der Zeichnung nicht dargestellt sind ggf. zusätzlich zu ergreifende Maßnahmen, wie etwa das Vorsehen von Entlüftungsbohrungen oder -nuten um das Verdrängen von Luft bei einem Spritzguss der Beschichtung 806 zu erleichten und so eine verbesserte Haftwirkung herbeizuführen.

Auch der Humeruskopf 306 aus Fig. 4 könnte so abgewandelt werden, dass auf die Nute 414 unterhalb der Artikulationsoberfläche 408 ganz oder teilweise verzichtet werden kann. Beispielsweise könnte ein Relief außerhalb der Artikulationsoberfläche 408 in einem Bereich 432 (vgl. Fig. 4) vorgesehen sein. Die Fläche 432 ist dem (verbleibenden, resektierten) Humerus zugewandt und reicht bis zum zurückversetzten Sockel 412, an den sich der Prothesenschaft 310 anschließt, vgl. Fig. 2. Die Fläche 432 umfasst den Untergrund für die Kappe 411 in einem Bereich bereit, in dem die Kappe 411 die äußere Kante 434 des Grundkörpers 402 umgreift, welche die Artikulationsoberfläche 408 begrenzt.

In dem Bereich 432 können bspw. Längs- und/oder Quernute vorhanden sein (in Fig. 4 nicht sichtbar), für deren Ausführung, Erstreckung, etc. sinngemäß vergleichbares gilt wie für die explizit in Fig. 4 dargestellten Nute 414, 416, 422 beschrieben. Ebenso ist es denkbar, zur Verbesserung der Haftwirkung Entlüftungsbohrungen und/oder -nute vorzusehen, und zwar entweder im Artikulationsbereich 408, auch wenn dort kein Relief vorgesehen sein sollte, oder im Bereich 432, oder in beiden Bereichen 408 und 432.

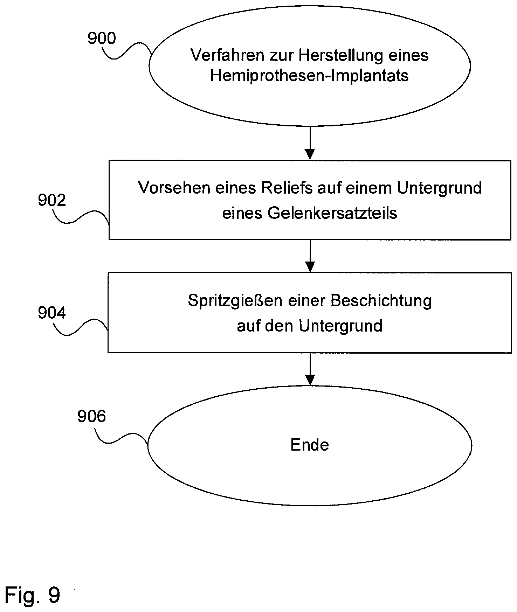

Fig. 9 zeigt ein Flussdiagramm mit einem Ausführungsbeispiel eines Verfahrens 900 zur Herstellung eines Hemiprothesen-Implantats. In Schritt 902 wird auf einem Untergrund eines Gelenkersatzteils zur verbesserten Anhaftung einer nachgelagert aufzubringenden Beschichtung ein Relief vorgesehen. Das Relief kann etwa bei der Herstellung eines Grundkörpers des Ersatzteils vorgesehen werden. So kann etwa auf oder an einem metallischen oder keramischen Körper mittels Gießen, Modellieren, Prägen, Pressen, etc. eine Reliefstruktur ausbildet werden. Zusätzlich oder alternativ kann nach einer Herstellung eines Grundkörpers eine nachgelagerte Bearbeitung erfolgen. So können bspw. in einen metallischen Körper durch zerspanende oder fräsende Bearbeitung Nute wie die in den Fig. 4, 6A gezeigten eingebracht werden. Ein aufgerauter Untergrund wie der in Fig. 5 angedeutet kann durch ein Strahlverfahren in einen metallischen Körper eingebracht werden.

In Schritt 904 wird die Beschichtung auf den vorbereiteten, d.h. mit einem Relief versehenen Untergrund aufgebracht. Bspw. kann ein Kunststoffmaterial wie ein TPE, bspw. PCU, mittels Spritzguss auf den Untergrund aufgebracht werden. Das Material umfließt die Reliefstrukturen. Nach einem Aushärten umschließt die Beschichtung erhabene Reliefstrukturen bzw. füllt vertiefte Reliefstrukturen aus. Die Beschichtung haftet dadurch in besonderer Weise am Grundkörper an.

Im Schritt 906 endet das Verfahren 900, bspw. in dem das hergestellte Hemiprothesen-Implantat in einen Implantatsatz integriert wird. Verfahren wie das Verfahren 900 und Abwandlungen davon ermöglichen eine kostengünstige Herstellung von Beschichtungen für standfeste Gelenkersatz-Hemiprothesen mit biokompatibler Artikulationsfläche.

Die Erfindung ist nicht auf die hier beschriebenen Ausführungsbeispiele und die darin hervorgehobenen Aspekte beschränkt. Vielmehr sind insbesondere aber nicht ausschließlich innerhalb des durch die anhängenden Ansprüche angegebenen Bereichs eine Vielzahl von Abwandlungen möglich, die im Rahmen fachmännischen Handelns liegen. Insbesondere sind dem Fachmann bestimmte Kombinationen von vorstehend separat beschriebenen Merkmalen als zweckmäßig oder vorteilhaft ersichtlich.

## Patentansprüche

1. Hemiprothesen-Implantat, mit
einem Gelenkersatzteil (108, 306) mit einer Artikulationsoberfläche (206, 408) zur Gleitpaarung mit einer Gelenkfläche eines natürlichen Gelenkgegenparts,
wobei die Artikulationsoberfläche (206, 408) mit einer auf einem Untergrund (204, 406) aufgebrachten Beschichtung (208, 410) ausgebildet ist,
wobei der Untergrund (204, 406) ein Relief (220, 428) zur Anhaftung der Beschichtung (208, 410) aufweist, wobei das Relief (220, 428) mindestens eine Vertiefung aufweist, und wobei mehrere Vertiefungen flächig zur Ausbildung von Bereichen mit einer Vielzahl von Vertiefungen angeordnet sind,
wobei die Vertiefungen muldenförmig ausgebildet sind,
**dadurch gekennzeichnet, dass**
unmittelbar an einem äußeren Sockel (730) eines den Untergrund (706) bildenden Grundkörpers (702) eine mindestens teilweise umlaufende Verankerungsnut (732) ausgeführt ist, und
dass das Material der Beschichtung (208, 410) weniger steif ist als das Material des Untergrunds (204, 406).

2. Implantat nach einem der vorhergehenden Ansprüche,
**dadurch gekennzeichnet, dass**
das Relief (220, 428) flächige Aussparungen aufweist, die kreisförmig und/oder halbmondförmig abgesenkte Bereiche bilden.

3. Implantat nach einem der vorhergehenden Ansprüche,
**dadurch gekennzeichnet, dass**
das Relief (428) eine Mehrzahl von Längsnuten (416) und/oder Quernuten (418) aufweist.

4. Implantat nach einem der vorhergehenden Ansprüche,
**dadurch gekennzeichnet, dass**
das Relief (620) eine Riffelung (621) und/oder eine Mehrzahl Zähne (626) aufweist.

5. Implantat nach einem der vorhergehenden Ansprüche,
**dadurch gekennzeichnet, dass**
in einer Vertiefung (710) des Reliefs eine in den Untergrund (702, 706) hineinführende Entlüftungsbohrung (716) und/oder eine von der Vertiefung (710) wegführende Entlüftungsnut (720) ausgeführt ist.

6. Implantat nach einem der vorhergehenden Ansprüche,
**dadurch gekennzeichnet, dass**
das Relief (518) mindestens einen aufgerauten Untergrundbereich (504) umfasst.

7. Implantat nach einem der vorhergehenden Ansprüche,
**dadurch gekennzeichnet, dass**
der Untergrund (204, 406) von einem Metall, einer Keramik, und/oder einem verstärkten Kunststoff gebildet wird.

8. Implantat nach einem der vorhergehenden Ansprüche,
**dadurch gekennzeichnet, dass**
das Material der Beschichtung (208, 410) ein thermoplastisches Elastomer, Polycarbonaturethan, ein Polyolefin-Polymer, ein Polyarylketon, und/oder ein Hydrogel umfasst.

9. Implantat nach einem der vorhergehenden Ansprüche,
**dadurch gekennzeichnet, dass**
ein Sockelbereich (740) eines den Untergrund (706) bildenden Grundkörpers (702) für den Übergang zur Artikulationsoberfläche (744) scharfkantig ausgeführt ist.

10. Implantat nach einem der vorhergehenden Ansprüche,
**dadurch gekennzeichnet, dass**
das Relief (818, 820) teilweise außerhalb der Artikulationsoberfläche (808, 814) ausgebildet ist.

11. Implantat nach einem der vorhergehenden Ansprüche,
**dadurch gekennzeichnet, dass**
das Implantat (108, 306) einen Humerushemiprothesenkopf, einen Hüfthemiprothesenkopf, eine Femurhemiprothesenkomponente, oder eine Sprungbeinhemiprothesenrolle umfasst.

12. Implantatsatz, umfassend
ein Basisimplantat (102, 302) zur Halterung eines Gelenkersatzteils;
ein Totalprothesen-Implantat mit einem ersten Gelenkersatzteil (104, 304) und mit einem zweiten Gelenkersatzteil (106), wobei das erste Gelenkersatzteil (104, 304) mit einer Artikulationsoberfläche zur Gleitpaarung mit einer Artikulationsoberfläche des zweiten Gelenkersatzteils (106) des Totalprothesen-Implantats versehen ist; und
ein Hemiprothesen-Implantat nach einem der vorhergehenden Ansprüche,
wobei das Gelenkersatzteil (108, 306) ein drittes Gelenkersatzteil bildet, und
wobei sowohl das erste (104, 304) als auch das dritte (108, 306) Gelenkersatzteil zur Halterung durch das Basisimplantat (102, 302) ausgebildet sind, so dass bei einer Implantation eine Totalprothese oder eine Hemiprothese implantiert werden kann.

13. Verfahren zur Herstellung eines Hemiprothesen-Implantats nach Anspruch 1, mit den folgenden Schritten:
Erzeugen (702) eines Reliefs mit mindestens einer Vertiefung auf einem Untergrund eines Gelenkersatzteils zur Anhaftung einer Beschichtung, wobei mehrere Vertiefungen flächig zur Ausbildung von Bereichen mit einer Vielzahl von Vertiefungen angeordnet werden, und wobei die Vertiefungen muldenförmig ausgebildet werden;
Erzeugen einer mindestens teilweise umlaufenden Verankerungsnut (732) unmittelbar an einem äußeren Sockel (730) eines den Untergrund (706) bildenden Grundkörpers (702);
Spritzgießen (704) der Beschichtung zur Ausbildung einer Artikulationsoberfläche des Gelenkersatzteils zur Gleitpaarung mit einer Gelenkfläche eines natürlichen Gelenkgegenparts.

## Claims

1. A hemiprosthesis implant, with
a replacement joint part (108, 306) with an articular surface (206, 408) for tribological pairing with a joint surface of a natural joint counterpart,
wherein the articular surface (206, 408) is embodied with a coating (208, 410) applied to a substrate (204, 406),
wherein the substrate (204, 406) comprises a relief (220, 428) for the adhesion of the coating (208, 410), wherein the relief (220, 428) comprises at least one indentation, and wherein several indentations are arranged two-dimensionally for the embodiment of regions with a plurality of indentations, wherein the indentations are embodied in the form of depressions,
**characterised in that**,
directly on an outer base (730) of a base body (702) forming the substrate (706), an at least partially circumferential anchoring groove (732) is embodied, and
that the material of the coating (208, 410) is less rigid than the material of the substrate (204, 406).

2. The implant according to any one of the preceding claims,
**characterised in that**
the relief (220, 428) comprises two-dimensionally arranged recesses, which form circular and/or crescent-shaped recessed regions.

3. The implant according to any one of the preceding claims,
**characterised in that**
the relief (428) comprises a multiplicity of longitudinal grooves (416) and/or transverse grooves (418).

4. The implant according to any one of the preceding claims,
**characterised in that**
the relief (620) comprises a fluting (621) and/or a multiplicity of teeth (626).

5. The implant according to any one of the preceding claims,
**characterised in that**,
in an indentation (710) of the relief, an air-vent borehole (716) leading into the substrate (702, 706) and/or an air-vent groove (720) leading away from the indentation (710) is embodied.

6. The implant according to any one of the preceding claims,
**characterised in that**
the relief (518) comprises at least one roughened substrate region (504).

7. The implant according to any one of the preceding claims,
**characterised in that**
the substrate (204, 406) is formed from a metal, a ceramic, and/or a reinforced synthetic material.

8. The implant according to any one of the preceding claims,
**characterised in that**
the material of the coating (208, 410) comprises a thermoplastic elastomer, polycarbonate urethane, a polyolefin polymer, a polyaryl ketone, and/or a hydrogel.

9. The implant according to any one of the preceding claims,
**characterised in that**
a base region (740) of a base body (702) forming the substrate (706) is embodied with a sharp edge for the transition to the articular surface (744).

10. The implant according to any one of the preceding claims,
**characterised in that**
the relief (818, 820) is embodied partially outside the articular surface (808, 814).

11. The implant according to any one of the preceding claims,
**characterised in that**
the implant (108, 306) comprises a humeral head hemiprosthesis, a hip head hemiprosthesis, a femoral hemiprosthesis component, or an ankle-joint trochlear hemiprosthesis.

12. An implant set, comprising
an implant base (102, 302) for the mounting of a replacement joint part;
a total prosthesis implant with a first replacement joint part (104, 304) and with a second replacement joint part (106), wherein the first replacement joint part (104, 304) is provided with an articular surface for tribological pairing with an articular surface of the second replacement joint part (106) of the total prosthesis implant; and
a hemiprosthesis implant according to any one of the preceding claims,
wherein the replacement joint part (108, 306) forms a third replacement joint part, and
wherein both the first (104, 304) and also the third (108, 306) replacement joint part are embodied for mounting through the implant base (102, 302), so that, in the case of an implantation, a total prosthesis or a hemiprosthesis can be implanted.

13. A method for the manufacture of a hemiprosthesis implant according to claim 1, with the following steps:
production (702) of a relief with at least one indentation on a substrate of a replacement joint part for the adhesion of a coating, wherein several indentations are arranged two-dimensionally for the embodiment of regions with a plurality of indentations, and wherein the indentations are embodied in the form of depressions;
production of an at least partially circumferential anchoring groove (732) directly on an outer base (730) of a base body (702) forming the substrate (706);
injection moulding (704) of the coating for the embodiment of an articular surface of the replacement joint part for tribological pairing with the joint surface of a natural joint counterpart.

## Revendications

1. Implant d'hémi-prothèse comportant :
une pièce de remplacement d'articulation (108, 306) avec une surface d'articulation (206, 408) pour un appariement avec une surface d'articulation d'une pièce complémentaire d'articulation naturelle,
dans lequel la surface d'articulation (206, 408) est réalisée avec un revêtement (208, 410) appliqué sur un substrat (204, 406),
dans lequel le substrat (204, 406) présente un relief (220, 428) pour l'adhérence du revêtement (208, 410), dans lequel le relief (220, 428) comporte au moins un évidement, et
dans lequel plusieurs évidements sont disposés à plat pour former des zones avec une pluralité d'évidements,
dans lequel les évidements sont réalisés sous forme de cuvettes, **caractérisé en ce que**
une rainure d'ancrage (732) au moins partiellement circonférentielle est réalisée directement sur un socle extérieur (730) d'un corps de base (702) formant le substrat (706), et
le matériau du revêtement (208, 410) est moins rigide que le matériau du substrat (204, 406).

2. Implant selon l'une des revendications précédentes, **caractérisé en ce que**
le relief (220, 428) comporte des cavités bidimensionnelles qui forment des zones abaissées de manière circulaire et/ou en demi-lune.

3. Implant selon l'une des revendications précédentes, **caractérisé en ce que**
le relief (428) comporte une pluralité de rainures longitudinales (416) et/ou de rainures transversales (418).

4. Implant selon l'une des revendications précédentes, **caractérisé en ce que**
le relief (620) comporte une cannelure (621) et/ou une pluralité de dents (626).

5. Implant selon l'une des revendications précédentes, **caractérisé en ce que**
un orifice d'aération (716) pénétrant dans le substrat (702, 706) et/ou une rainure d'aération (720) s'éloignant de l'évidement (710) est réalisé dans un évidement (710) du relief.

6. Implant selon l'une des revendications précédentes, **caractérisé en ce que**
le relief (518) comprend au moins une zone de substrat rugueuse (504).

7. Implant selon l'une des revendications précédentes, **caractérisé en ce que**
le substrat (204, 406) est formé à partir d'un métal, d'une céramique et/ou d'une matière plastique renforcée.

8. Implant selon l'une des revendications précédentes, **caractérisé en ce que**
le matériau du revêtement (208, 410) comprend un élastomère thermoplastique, un polycarbonate-uréthane, un polymère de polyoléfine, une cétone polyaryle et/ou un hydrogel.

9. Implant selon l'une des revendications précédentes, **caractérisé en ce que**
une zone de socle (740) d'un corps de base (702) formant le substrat (706) est réalisée avec des arêtes vives pour la transition vers la surface d'articulation (744).

10. Implant selon l'une des revendications précédentes,
**caractérisé en ce que**
le relief (818, 820) est formé en partie à l'extérieur de la surface d'articulation (808, 814).

11. Implant selon l'une des revendications précédentes,
**caractérisé en ce que**
l'implant (108, 306) comprend une hémi-prothèse de tête d'humérus, une hémi-prothèse de tête de hanche, une hémi-prothèse de composant de fémur ou une hémi-prothèse d'astragale.

12. Jeu d'implants comportant :
un implant de base (102, 302) pour maintenir une pièce de remplacement d'articulation ;
un implant de prothèse totale avec une première pièce de remplacement d'articulation (104, 304) et une deuxième pièce de remplacement d'articulation (106), la première pièce de remplacement d'articulation (104, 304) étant pourvue d'une surface d'articulation pour l'appariement à une surface d'articulation de la deuxième pièce de remplacement d'articulation (106) de l'implant de prothèse totale ; et
un implant d'hémi-prothèse selon l'une des revendications précédentes,
dans lequel la pièce de remplacement d'articulation (108, 306) forme une troisième pièce de remplacement d'articulation, et
dans lequel aussi bien la première (104, 304) que la troisième (108, 306) pièce de remplacement d'articulation sont conçues pour un maintien à travers l'implant de base (102, 302), de sorte qu'une prothèse totale ou une hémi-prothèse peut être implantée lors d'une implantation.

13. Procédé pour fabriquer un implant d'hémi-prothèse selon la revendication 1, comportant les étapes suivantes consistant à :
générer (702) un relief avec au moins un évidement sur un substrat d'une pièce de remplacement d'articulation pour l'adhérence d'un revêtement, dans lequel plusieurs évidements sont agencés de manière bidimensionnelle pour former des zones avec une pluralité d'évidements, et dans lequel les évidements sont réalisés sous forme de cuvettes ;
générer une rainure d'ancrage (732) s'étendant au moins partiellement directement sur un socle extérieur (730) d'un corps de base (702) formant le substrat (706) ;
mouler par injection (704) le revêtement pour former une surface d'articulation de la pièce de remplacement d'articulation pour un appariement à une surface d'articulation d'une pièce complémentaire d'articulation naturelle.
